# EUROPEAN PATENT APPLICATION

(11) **EP 2 048 162 A1**
(43) Date of publication of application: **15.04.2009**
(21) Application number: 07805903.7
(22) Date of filing: 07.08.2007
(51) Int. Cl.: C07K 16/28, A61K 39/395, A61P 3/10, A61P 5/48, A61P 15/00, A61P 25/02, A61P 25/18, A61P 25/24, A61P 43/00, C12N 15/09, C12Q 1/02, C40B 30/06, G01N 33/15, G01N 33/50, G01N 33/53, C12P 21/08

(54) **NOVEL MONOCLONAL ANTIBODY AND USE OF THE SAME**

(30) Priority: 08.08.2006 JP 2006216282
(71) Applicant: President, Kyoto University, Kyoto 606-8501 (JP); Takeda Pharmaceutical Company Limited, Osaka-shi, Osaka 541-0045 (JP)
(72) Inventor: SHIRAKAWA, Masahiro, Kyoto-shi, Kyoto 615-8530 (JP); INOOKA, Hiroshi, Tsukuba-shi, Ibaraki 300-4293 (JP)
(74) Representative: Helbing, Jörg
(86) International application number: PCT/JP2007/065465
(87) International publication number: WO 2008/018472

(57) **Abstract**

The present invention provides an anti-PAC1 monoclonal antibody capable of recognizing a PAC1 having a native structure, a PAC1 activity regulator (in particular, activity inhibitor) containing the antibody, a prophylactic/therapeutic agent for a disease associated with accentuation of a bioactivity of PAC1, containing the antibody, a diagnostic reagent for a disease associated with an abnormality of PAC1 activity, containing the antibody, and a screening method for a substance that regulates the expression of PAC1, using the antibody and a PAC1-expressing cell.

SEQUENCE LISTING

<110>  Kyoto University
       Takeda Pharmaceutical Company Limited

<120>  NOVEL MONOCLONAL ANTIBODY AND USE OF THE SAME

<130>  091104

<150>  JP 2006-216282 @
<151>  2006-08-08

<160>  2

<170>  PatentIn version 3.3

<210>  1
<211>  1404
<212>  DNA
<213>  Homo sapiens


<220>
<221>  CDS
<222>  (1)..(1404)

<220>
<221>  sig_peptide
<222>  (1)..(60)

<220>
<221>  mat_peptide
<222>  (61)..(1404)

<400>  1
atg gct ggt gtc gtg cac gtt tcc ctg gct gct ctc ctc ctg ctg cct       48
Met Ala Gly Val Val His Val Ser Leu Ala Ala Leu Leu Leu Leu Pro
-20                 -15                 -10                 -5

atg gcc cct gcc atg cat tct gac tgc atc ttc aag aag gag caa gcc       96
Met Ala Pro Ala Met His Ser Asp Cys Ile Phe Lys Lys Glu Gln Ala
            -1  1               5                   10

atg tgc ctg gag aag atc cag agg gcc aat gag ctg atg ggc ttc aat      144
Met Cys Leu Glu Lys Ile Gln Arg Ala Asn Glu Leu Met Gly Phe Asn
        15                  20                  25

gat tcc tct cca ggc tgt cct ggg atg tgg gac aac atc acg tgt tgg      192
Asp Ser Ser Pro Gly Cys Pro Gly Met Trp Asp Asn Ile Thr Cys Trp
    30                  35                  40

aag ccc gcc cat gtg ggt gag atg gtc ctg gtc agc tgc cct gag ctc      240
Lys Pro Ala His Val Gly Glu Met Val Leu Val Ser Cys Pro Glu Leu
45                  50                  55                  60

ttc cga atc ttc aac cca gac caa gtc tgg gag acc gaa acc att gga      288
Phe Arg Ile Phe Asn Pro Asp Gln Val Trp Glu Thr Glu Thr Ile Gly
                65                  70                  75

gag tct gat ttt ggt gac agt aac tcc tta gat ctc tca gac atg gga      336
Glu Ser Asp Phe Gly Asp Ser Asn Ser Leu Asp Leu Ser Asp Met Gly
            80                  85                  90

gtg gtg agc cgg aac tgc acg gag gat ggc tgg tcg gaa ccc ttc cct 384
Val Val Ser Arg Asn Cys Thr Glu Asp Gly Trp Ser Glu Pro Phe Pro
95 100 105

cat tac ttt gat gcc tgt ggg ttt gat gaa tat gaa tct gag act ggg 432
His Tyr Phe Asp Ala Cys Gly Phe Asp Glu Tyr Glu Ser Glu Thr Gly
110 115 120

gac cag gat tat tac tac ctg tca gtg aag gcc ctc tac acg gtt ggc 480
Asp Gln Asp Tyr Tyr Tyr Leu Ser Val Lys Ala Leu Tyr Thr Val Gly
125 130 135 140

tac agc aca tcc ctc gtc acc ctc acc act gcc atg gtc atc ctt tgt 528
Tyr Ser Thr Ser Leu Val Thr Leu Thr Thr Ala Met Val Ile Leu Cys
145 150 155

cgc ttc cgg aag ctg cac tgc aca cgc aac ttc atc cac atg aac ctg 576
Arg Phe Arg Lys Leu His Cys Thr Arg Asn Phe Ile His Met Asn Leu
160 165 170

ttt gtg tcg ttc atg ctg agg gcg atc tcc gtc ttc atc aaa gac tgg 624
Phe Val Ser Phe Met Leu Arg Ala Ile Ser Val Phe Ile Lys Asp Trp
175 180 185

att ctg tat gcg gag cag gac agc aac cac tgc ttc atc tcc act gtg 672
Ile Leu Tyr Ala Glu Gln Asp Ser Asn His Cys Phe Ile Ser Thr Val
190 195 200

gaa tgt aag gcc gtc atg gtt ttc ttc cac tac tgt gtt gtg tcc aac 720
Glu Cys Lys Ala Val Met Val Phe Phe His Tyr Cys Val Val Ser Asn
205 210 215 220

tac ttc tgg ctg ttc atc gag ggc ctg tac ctc ttc act ctg ctg gtg 768
Tyr Phe Trp Leu Phe Ile Glu Gly Leu Tyr Leu Phe Thr Leu Leu Val
225 230 235

gag acc ttc ttc cct gaa agg aga tac ttc tac tgg tac acc atc att 816
Glu Thr Phe Phe Pro Glu Arg Arg Tyr Phe Tyr Trp Tyr Thr Ile Ile
240 245 250

ggc tgg ggg acc cca act gtg tgt gtg aca gtg tgg gct acg ctg aga 864
Gly Trp Gly Thr Pro Thr Val Cys Val Thr Val Trp Ala Thr Leu Arg
255 260 265

ctc tac ttt gat gac aca ggc tgc tgg gat atg aat gac agc aca gct 912
Leu Tyr Phe Asp Asp Thr Gly Cys Trp Asp Met Asn Asp Ser Thr Ala
270 275 280

ctg tgg tgg gtg atc aaa ggc cct gtg gtt ggc tct atc atg gtt aac 960
Leu Trp Trp Val Ile Lys Gly Pro Val Val Gly Ser Ile Met Val Asn
285 290 295 300

ttt gtg ctt ttt att ggc att atc gtc atc ctt gtg cag aaa ctt cag 1008
Phe Val Leu Phe Ile Gly Ile Ile Val Ile Leu Val Gln Lys Leu Gln
305 310 315

tct cca gac atg gga ggc aat gag tcc agc atc tac ttg cga ctg gcc 1056
Ser Pro Asp Met Gly Gly Asn Glu Ser Ser Ile Tyr Leu Arg Leu Ala
320 325 330

cgg tcc acc ctg ctg ctc atc cca cta ttc gga atc cac tac aca gta 1104
Arg Ser Thr Leu Leu Leu Ile Pro Leu Phe Gly Ile His Tyr Thr Val
335 340 345

ttt gcc ttc tcc cca gag aat gtc agc aaa agg gaa aga ctc gtg ttt      1152
Phe Ala Phe Ser Pro Glu Asn Val Ser Lys Arg Glu Arg Leu Val Phe
    350                 355                 360

gag ctg ggg ctg ggc tcc ttc cag ggc ttt gtg gtg gct gtt ctc tac      1200
Glu Leu Gly Leu Gly Ser Phe Gln Gly Phe Val Val Ala Val Leu Tyr
365                 370                 375                 380

tgt ttt ctg aat ggt gag gta caa gcg gag atc aag cga aaa tgg cga      1248
Cys Phe Leu Asn Gly Glu Val Gln Ala Glu Ile Lys Arg Lys Trp Arg
                385                 390                 395

agc tgg aag gtg aac cgt tac ttc gct gtg gac ttc aag cac cga cac      1296
Ser Trp Lys Val Asn Arg Tyr Phe Ala Val Asp Phe Lys His Arg His
            400                 405                 410

ccg tct ctg gcc agc agt ggg gtg aat ggg ggc acc cag ctc tcc atc      1344
Pro Ser Leu Ala Ser Ser Gly Val Asn Gly Gly Thr Gln Leu Ser Ile
        415                 420                 425

ctg agc aag agc agc tcc caa atc cgc atg tct ggc ctc cct gct gac      1392
Leu Ser Lys Ser Ser Ser Gln Ile Arg Met Ser Gly Leu Pro Ala Asp
    430                 435                 440

aat ctg gcc acc                                                      1404
Asn Leu Ala Thr
445


<210>  2
<211>  468
<212>  PRT
<213>  Homo sapiens

<400>  2

Met Ala Gly Val Val His Val Ser Leu Ala Ala Leu Leu Leu Leu Pro
-20                 -15                 -10                 -5


Met Ala Pro Ala Met His Ser Asp Cys Ile Phe Lys Lys Glu Gln Ala
            -1  1               5                   10


Met Cys Leu Glu Lys Ile Gln Arg Ala Asn Glu Leu Met Gly Phe Asn
        15                  20                  25


Asp Ser Ser Pro Gly Cys Pro Gly Met Trp Asp Asn Ile Thr Cys Trp
    30                  35                  40


Lys Pro Ala His Val Gly Glu Met Val Leu Val Ser Cys Pro Glu Leu
45                  50                  55                  60


Phe Arg Ile Phe Asn Pro Asp Gln Val Trp Glu Thr Glu Thr Ile Gly
                65                  70                  75


Glu Ser Asp Phe Gly Asp Ser Asn Ser Leu Asp Leu Ser Asp Met Gly
            80                  85                  90

Val Val Ser Arg Asn Cys Thr Glu Asp Gly Trp Ser Glu Pro Phe Pro
        95                  100                 105

His Tyr Phe Asp Ala Cys Gly Phe Asp Glu Tyr Glu Ser Glu Thr Gly
    110                 115                 120

Asp Gln Asp Tyr Tyr Tyr Leu Ser Val Lys Ala Leu Tyr Thr Val Gly
125                 130                 135                 140

Tyr Ser Thr Ser Leu Val Thr Leu Thr Thr Ala Met Val Ile Leu Cys
                145                 150                 155

Arg Phe Arg Lys Leu His Cys Thr Arg Asn Phe Ile His Met Asn Leu
            160                 165                 170

Phe Val Ser Phe Met Leu Arg Ala Ile Ser Val Phe Ile Lys Asp Trp
        175                 180                 185

Ile Leu Tyr Ala Glu Gln Asp Ser Asn His Cys Phe Ile Ser Thr Val
    190                 195                 200

Glu Cys Lys Ala Val Met Val Phe Phe His Tyr Cys Val Val Ser Asn
205                 210                 215                 220

Tyr Phe Trp Leu Phe Ile Glu Gly Leu Tyr Leu Phe Thr Leu Leu Val
                225                 230                 235

Glu Thr Phe Phe Pro Glu Arg Arg Tyr Phe Tyr Trp Tyr Thr Ile Ile
            240                 245                 250

Gly Trp Gly Thr Pro Thr Val Cys Val Thr Val Trp Ala Thr Leu Arg
        255                 260                 265

Leu Tyr Phe Asp Asp Thr Gly Cys Trp Asp Met Asn Asp Ser Thr Ala
    270                 275                 280

Leu Trp Trp Val Ile Lys Gly Pro Val Val Gly Ser Ile Met Val Asn
285                 290                 295                 300

Phe Val Leu Phe Ile Gly Ile Ile Val Ile Leu Val Gln Lys Leu Gln
                305                 310                 315

Ser Pro Asp Met Gly Gly Asn Glu Ser Ser Ile Tyr Leu Arg Leu Ala
            320                 325                 330

Arg Ser Thr Leu Leu Leu Ile Pro Leu Phe Gly Ile His Tyr Thr Val

335                 340                 345

Phe Ala Phe Ser Pro Glu Asn Val Ser Lys Arg Glu Arg Leu Val Phe
    350                 355                 360

Glu Leu Gly Leu Gly Ser Phe Gln Gly Phe Val Val Ala Val Leu Tyr
365                 370                 375                 380

Cys Phe Leu Asn Gly Glu Val Gln Ala Glu Ile Lys Arg Lys Trp Arg
                385                 390                 395

Ser Trp Lys Val Asn Arg Tyr Phe Ala Val Asp Phe Lys His Arg His
            400                 405                 410

Pro Ser Leu Ala Ser Ser Gly Val Asn Gly Gly Thr Gln Leu Ser Ile
        415                 420                 425

Leu Ser Lys Ser Ser Ser Gln Ile Arg Met Ser Gly Leu Pro Ala Asp
    430                 435                 440

Asn Leu Ala Thr
445

## Description

### [Technical Field]

The present invention relates to a plurality of novel antibodies against pituitary adenylate cyclase activating polypeptide type 1 receptor (hereinafter abbreviated as "PAC1")) with different antigen determinants, and use thereof.

### [Background Art]

PAC1 is a receptor of pituitary adenylate cyclase activating polypeptide (hereinafter abbreviated as "PACAP"), and is one of 7-transmembrane type G protein coupled receptors (GPCRs). PACAP is known to exhibit various bioactivities in the central and peripheral nervous systems by binding to PAC1. Hence, it has been found that disturbances in the PACAP-PAC1 signal pathway are associated with depression, mental behavioral dysfunction, sexual dysfunction, circadian rhythm photosynchronization disorder and the like in the central nervous system, and are also associated with insulin secretion disorders such as diabetes and hyperinsulinemia and the like in the periphery. This means that PAC1 can be a drug target for the treatment of the foregoing diseases and disorders.

Because membrane proteins, represented by GPCRs, are targets of drug discovery, elucidation of the structures and functions thereof is important. Conformational analysis of a protein necessitates crystallization of the protein; however, because the hydrophobic region of a membrane protein is covered by a detergent that solubilizes the same, the protein area that can contribute to crystal formation is limited, so it is quite difficult to obtain a crystal of high quality. For PAC1 as well, no good crystals have been obtained so far that permit conformational analysis.

Aiming at facilitating the crystallization of a membrane protein, attempts have been made to increase the size of the hydrophilic moiety thereof by means of an antibody specifically binding to the membrane protein, so as to allow the hydrophilic part to produce an intermolecular interaction sufficient to form crystal lattices. However, to perform this technique, a monoclonal antibody capable of recognizing the hydrophilic part of the desired membrane protein, and recognizing a structure by which the membrane protein is capable of exhibiting the essential physiological function thereof (native structure), is essential. As a means of acquiring an antibody capable of recognizing the native structure of a membrane protein, a method is performed wherein an animal is immunized using as an antigen a cell that expresses the membrane protein, or a membrane fraction thereof, as is; however, because the amount of GPCR expressed on the cell membrane surface is small, it is very unlikely that the desired antibody is obtained. For PAC1, all the antibodies that have been reported so far (non-patent documents 1 to 3) are only capable of recognizing a PAC1 having a denatured, non-physiological structure.

As stated above, PAC1 is a receptor of PACAP, which is widely distributed in the central nervous system to the peripheral nerves, and exhibits a broad range of bioactivities; therefore, an antibody capable of recognizing a native structure of PAC1, as a substance that binds to PAC1 to regulate the bioactivity of PACAP, can be a candidate for a pharmaceutical by itself. Antibodies have excellent properties of low prevalence of side effects and long sustainability of efficacy for pharmaceutical products because of the high specificity and long blood half-life thereof. Because PAC1 is a cell surface receptor, an anti-PAC1 antibody for therapeutic use is desirably one that recognizes an extracellular region of PAC1, and any publicly known anti-PAC1 antibodies recognize a C-terminal region on the cytoplasmic side.
Therefore, there is a strong demand for an anti-PAC1 monoclonal antibody capable of recognizing an extracellular region of PAC1 having a native structure.

As another means of acquiring an antibody capable of recognizing a native structure of a membrane protein, a method
wherein a purified membrane protein reference standard is used as an antigen is likely. As stated above, to purify a membrane protein while retaining the native structure thereof, solubilization with a detergent is required. It has already been reported that PAC1 was solubilized using digitonin, and purified (non-patent document 4). However, the purified PAC1 obtained by that method has the hydrophobic region thereof covered by the detergent, so the portion capable of serving as an antigen determinant is highly limited; therefore, it is unsuitable as an antigen. Hence, the same problem as that faced in crystallization makes it difficult to acquire an antibody capable of recognizing the native structure of PAC1.
[non-patent document 1]Rong-Ming Lyu et al., J. Biol. Chem., (US), vol. 275, pp. 36134-36142, 2000
[non-patent document 2]Solveig Schulz et al., Clin. Cancer Res., (US), vol. 10, pp. 8235-8242, 2004
[non-patent document 3]S.T. Anderson et al., J. Neuroendocrinol., (US), vol. 17, pp. 298-305, 2005
[non-patent document 4]Tetsuya Ohtaki et al., J. Biol. Chem., (US), vol. 273, pp. 15464-15473, 1998

### [Disclosure of the Invention]

### [Problems to Be Solved by the Invention]

The present invention is intended to provide a novel anti-PAC1 antibody capable of recognizing human PAC1 possessing a native structure, to provide a prophylactic/therapeutic agent or diagnostic reagent for a disease associated with an abnormality of PAC1 activity using the antibody, and to provide a method of screening for a substance that regulates the expression of PAC1.

### [Means of Solving the Problems]

The present inventors extensively investigated the choice and blending ratio of the detergent used to solubilize PAC1, and the like, and succeeded in obtaining a purified PAC1 suitable as an antigen for preparing an antibody. Furthermore, the present inventors succeeded in preparing a plurality of mouse anti-human PAC1 monoclonal antibodies with different properties that recognize an extracellular region of human PAC1 having a native structure.
The present inventors conducted further investigations based on these findings, and developed the present invention.

Accordingly, the present invention provides:
[1] an anti-PAC1 monoclonal antibody capable of recognizing a PAC1 having a native structure;
[2] the antibody described in [1] above, which recognizes an extracellular region of PAC1;
[3] the antibody described in [2] above, which inhibits the binding of PAC1 to PACAP;
[4] the antibody described in [3] above, which inhibits a bioactivity of PAC1;
[5] the antibody described in [2] above, which does not inhibit the binding of PAC1 to PACAP;
[6] the antibody described in [5] above, which promotes the binding of another anti-PAC1 monoclonal antibody to PAC1;
[7] the antibody described in [6] above, wherein the other anti-PAC1 monoclonal antibody inhibits the binding of PAC1 to PACAP;
[8] the antibody described in [1] above, which is further capable of recognizing denatured PAC1;
[9] a PAC1 activity regulator comprising the antibody described in [1] above;
[10] a PAC1 activity inhibitor comprising the antibody described in [4] above;
[11] the agent described in [10] above, which is to be used for the prevention/treatment of a disease associated with accentuation of a bioactivity of PAC1;
[12] a PAC1 detection agent comprising the antibody described in [1] above;
[13] the agent described in [12] above, which is to be used for the diagnosis of a disease associated with an abnormality of PAC1 activity;
[14] a screening method for a substance that regulates the expression of PAC1, comprising using the antibody described in [1] above and a PAC1-expressing cell; and the like.

### [Effect of the Invention]

The novel anti-PAC1 antibodies of the present invention are capable of recognizing an extracellular region of PAC1 having a native structure, and are hence capable of binding to PAC1 in vivo, and regulating the activity thereof; therefore, the antibodies are useful as prophylactic/therapeutic agents for diseases associated with an abnormality of PAC1 activity, for example, depression, mental behavioral dysfunction, sexual dysfunction, circadian rhythm photosynchronization disorder and the like, and as diagnostic reagents for the foregoing diseases and insulin secretion disorders such as diabetes and hyperinsulinemia and the like.

### [Brief Description of the Drawings]

[FIG. 1] A graph showing the apparent binding affinities of Sf9 cell samples and PACAP before and after membrane fraction solubilization using an SM1200/CHS mixed micelle system.
[FIG. 2] Graphs showing Scatchard analyses of a membrane fraction (A) and a membrane fraction solubilizing solution (B).
[FIG. 3] A graph showing the binding affinities of membrane fraction solubilizing solutions for PACAP.
[FIG. 4] A graph showing the binding affinity for PACAP of each eluted fraction obtained by affinity adsorption.
[FIG. 5] Graphs showing the binding affinity for PACAP of each eluted fraction obtained by hydroxyapatite chromatography (A), and the apparent binding affinities for PACAP of the Main fraction, Sub1 fraction and Sub2 fraction obtained by hydroxyapatite chromatography (B).
[FIG. 6] Graphs showing Scatchard analyses of a Main fraction obtained by hydroxyapatite chromatography (A) and an eluted fraction obtained by affinity chromatography (B).
[FIG. 7] Graphs showing the binding affinities for antigen PAC1 receptor of mouse anti-human PAC1 monoclonal IgG antibodies obtained in Example 2 (A) and Fab fragments thereof (B).
[FIG. 8] Graphs showing the inhibition of the binding of PACAP to PAC1 by mouse anti-human PAC1 monoclonal IgG antibodies obtained in Example 2 (A) and Fab fragments thereof (B).
[FIG. 9-1] Graphs showing the inhibition of the binding activation of GTP-γS to PAC1 by mouse anti-human PAC1 monoclonal IgG antibodies obtained in Example 2 (A to C).
[FIG. 9-2] Graphs showing the inhibition of the binding activation of GTP-γS to PAC1 by mouse anti-human PAC1 monoclonal IgG antibodies obtained in Example 2 (D, E).
[FIG. 10-1] Graphs showing results of competitive ELASA (direct adsorption method) using two different kinds of mouse anti-human PAC1 monoclonal IgG antibodies obtained in Example 2 (A to F).
[FIG. 10-2] Graphs showing results of competitive ELASA (direct adsorption method) using two different kinds of mouse anti-human PAC1 monoclonal IgG antibodies obtained in Example 2 (G to L).
[FIG. 10-3] Graphs showing results of competitive ELASA (direct adsorption method) using two different kinds of mouse anti-human PAC1 monoclonal IgG antibodies obtained in Example 2 (M to R).
[FIG. 10-4] Graphs showing results of competitive ELASA (direct adsorption method) using two different kinds of mouse anti-human PAC1 monoclonal IgG antibodies obtained in Example 2 (S to V).

The present invention provides novel anti-PAC1 antibodies capable of recognizing a PAC1 having a native structure (hereinafter also referred to as "antibodies of the present invention"). Here, "a native structure" means any structure PAC1 can assume in a condition where it is capable of exhibiting the essential physiological function thereof, i.e., in a condition where it occurs naturally in the cell membrane of a living organism. Because PAC1 is a GPCR, the structure thereof changes between the active state (i.e., a state bound with PACAP or another PAC1 agonist) and the inactive state (a state not bound with a ligand, or a state bound with an antagonist), but the structure in any state corresponds to "a native structure" in the present invention.

An antibody of the present invention may have any portion of PAC1 as the antigen determinant thereof, as far as it is capable of recognizing a PAC1 having a native structure; however, because a purified PAC1/detergent complex is preferably used as an immunogen, the antibody of the present invention is preferably one that recognizes any one of the hydrophilic regions of PAC1 (i.e., N-terminal region, extracellular 1st to 3rd loop regions, cytoplasmic 1st to 3rd loop regions, C-terminal region), more preferably recognizes any one of the extracellular regions (i.e., N-terminal region, extracellular 1st to 3rd loop regions). Because an antibody of the present invention that recognizes an extracellular region is capable of binding to PAC1 expressed on the surface of intact cells, it is particularly advantageous in use as an antibody pharmaceutical.

Although the isotype of the antibody used in the present invention is not subject to limitation, it is preferably IgG, IgM or IgA, particularly preferably IgG.

The antibody used in the present invention is not subject to limitation, as long as it has at least a complementality determining region (CDR) for specifically recognizing and binding to the target antigen; in addition to the whole antibody molecule, the antibody may, for example, be a fragment such as Fab, Fab', or F(ab')₂, Fv, V_{H}, a genetically engineered conjugate molecule such as scFv, scFv-Fc, dsFv, minibody, or diabody, or a derivative thereof modified with a molecule having protein stabilizing action, such as polyethylene glycol (PEG), or the like, and the like.

An antibody against PAC1 can be produced by a method of antibody or antiserum production known per se.

Hereinafter, a method of preparing an immunogen for an antibody of the present invention, and a method of producing the antibody are described.

### (1) Preparation of antigen

As an antigen used to prepare an antibody of the present invention, any of PAC1 or a partial peptide thereof, a (synthetic) peptide having 1 kind or 2 kinds or more of the same antigen determinant thereas, and the like, can be used.

PAC1 or a partial peptide thereof is produced by, for example, (a) preparation from a tissue or cell of a warm-blooded animal such as a human, monkey, rat, mouse, or chicken using a publicly known method or a method thereon, (b) chemical synthesis by a publicly known method of peptide synthesis using a peptide synthesizer and the like, (c) cultivation of a transformant containing a DNA that encodes PAC1 or a partial peptide thereof, or (d) biochemical synthesis using a cell-free transcription/translation system with a nucleic acid that encodes PAC1 or a partial peptide thereof as the template.

The PAC1 protein to be used in the present invention is a protein comprising the same or substantially the same amino acid sequence as an amino acid sequence shown by SEQ ID NO: 2.
PAC1 may be a protein isolated and purified from, for example, a cell (e.g., hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell, fibroblast, fibrocyte, myocyte, adipocyte, immunocyte (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, interstitial cell, or a corresponding precursor cell, stem cell or cancer cell thereof, and the like) of a mammal (e.g., human, mouse, rat, rabbit, sheep, swine, bovine, horse, cat, dog, monkey, chimpanzee and the like), or any tissue or organ in which these cells are present [e.g., brain or any portion of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gallbladder, bone marrow, adrenal gland, skin, lung, gastrointestinal tract (e.g., large intestine and small intestine), blood vessel, heart, thymus, spleen, submandibular gland, peripheral blood, prostate, testicle, ovary, placenta, uterus, bone, joint, adipose tissue (e.g., brown adipose tissue, white adipose tissue), skeletal muscle and the like] and the like. The PAC1 protein may also be a biochemically synthesized protein or a protein synthesized using a cell-free translation system. Alternatively, the PAC1 protein may be a recombinant protein produced by a transformant introduced with a nucleic acid having the base sequence that encodes the above-described amino acid sequence.

As "substantially the same amino acid sequence" as an amino acid sequence shown by SEQ ID NO: 2, an amino acid sequence having a homology of about 60% or more, preferably about 70% or more, more preferably about 80% or more, and particularly preferably about 90% or more, with an amino acid sequence shown by SEQ ID NO: 2 can be mentioned. Here, the "homology" means a ratio (%) of identical amino acid residues and similar amino acid residues to all overlapping amino acid residues in the best alignment where two amino acid sequences are aligned using a mathematical algorithm known in the technical field (preferably, the algorithm considers introduction of gaps on one or both sides of the sequence for the best alignment). "A similar amino acid" means an amino acid having similar physiochemical properties; examples thereof include amino acids classified under the same group, such as aromatic amino acids (Phe, Trp, Tyr), aliphatic amino acids (Ala, Leu, Ile, Val), polar amino acids (Gln, Asn), basic amino acids (Lys, Arg, His), acidic amino acids (Glu, Asp), amino acids having a hydroxyl group (Ser, Thr) and amino acids having a small side-chain (Gly, Ala, Ser, Thr, Met). Substitution by such similar amino acids is expected to give no change in the phenotype of protein (i.e., constitutive amino acid substitution). Specific examples of constitutive amino acid substitution are obvious in the relevant technical field, and are described in various documents (see, for example, Bowie et al., Science, 247:1306-1310 (1990)).
Homology of the amino acid sequences in the present specification can be calculated under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool). As other algorithm for determining the homology of amino acid sequence, for example, the algorithm described in Karlin et al., Proc. Matl. Acad. Sci. USA, 90: 5873-5877 (1993) [the algorithm is incorporated in NBLAST and XBLAST program (version 2.0) (Altschul et al., Nucleic Acids Res., 25: 3389-3402 (1997))], the algorithm described in Needleman et al., J. Mol. Biol., 48: 444-453 (1970) [the algorithm is incorporated in the GAP program in GCG software package], the algorithm described in Myers and Miller, CABIOS, 4: 11-17 (1988) [the algorithm is incorporated in ALIGN program (version 2.0) which is a part of the CGC sequence alignment software package], the algorithm described in Pearson et al., Proc. Natl. Acad. Sci. USA, 85: 2444-2448 (1988) [the algorithm is incorporated in the FASTA program in GCG software package] and the like can be mentioned, and they can be preferably used in a similar way.
More preferably, the substantially the same amino acid sequence with an amino acid sequence shown by SEQ ID NO: 2 is an amino acid sequence having an identity of about 60% or more, preferably about 70% or more, more preferably about 80% or more, particularly preferably about 90%, with an amino acid sequence shown by SEQ ID NO: 2.

As examples of a protein comprising substantially the same amino acid sequence as the amino acid sequence shown by SEQ ID NO:2, a protein comprising substantially the same amino acid sequence as the aforementioned amino acid sequence shown by SEQ ID NO:2, and having substantially the same quality of activity as a protein comprising the amino acid sequence shown by SEQ ID NO:2 and the like are preferable.

As examples of substantially the same quality of activity, ligand (i.e., PACAP) binding activity, signal transduction activities (for example, adenylate cyclase stimulation activity (increased cAMP production), activity for GTP derivative binding to conjugated trimeric G protein, phospholipase C stimulation activity (increased intracellular Ca concentration) and the like) and the like can be mentioned. "Substantially the same quality" means that the activities are qualitatively (for example, physiologically or pharmacologically) equivalent to each other. Therefore, it is preferable that the activities such as ligand binding activity and signal transduction activity be equivalent to each other (for example, about 0.5 to 2 times), but the quantitative factors of these activities, such as the extent of activity and the molecular weight of the protein, may be different.
Measurements of ligand binding activity and signal transduction activity can be performed in accordance with methods known per se (see, for example, WO 03/055507).

Examples of PAC1 used in the present invention include proteins having (1) an amino acid sequence resulting from deletion of 1 or 2 or more (preferably about 1 to 100, preferably about 1 to 50, more preferably about 1 to 10, particularly preferably one to several (2, 3, 4 or 5)), amino acids in the amino acid sequence shown by SEQ ID NO:2, (2) an amino acid sequence resulting from addition of 1 or 2 or more (preferably about 1 to 100, preferably about 1 to 50, more preferably about 1 to 10, particularly preferably one to several (2, 3, 4 or 5)), amino acids in the amino acid sequence shown by SEQ ID NO:2, (3) an amino acid sequence resulting from insertion of 1 or 2 or more (preferably about 1 to 50, preferably about 1 to 10, more preferably one to several (2, 3, 4 or 5)), amino acids in the amino acid sequence shown by SEQ ID NO:2, (4) an amino acid sequence resulting from substitution of 1 or 2 or more (preferably about 1 to 50, preferably about 1 to 10, more preferably one to several (2, 3, 4 or 5)), amino acids in the amino acid sequence shown by SEQ ID NO:2 by other amino acids, or (5) an amino acid sequence being a combination thereof, and the like.
When an amino acid sequence is inserted, deleted or substituted as described above, the position of the insertion, deletion or substitution is not particularly limited, as far as the activity of the protein is retained.

The PAC1 used in the present invention is preferably the human PAC1 protein having the amino acid sequence shown by SEQ ID NO:2 (GenBank accession number: NP_001109), or an orthologue thereof in another mammal [for example, mouse and rat orthologues registered under accession numbers NP_031433 and NP_598195, respectively, in the GenBank (having identities of about 87.5% and about 87.3%, respectively, to human PAC1) and the like].

For the proteins and peptides described in the present specification, the left end indicates the N-terminus (amino terminus) and the right end indicates the C-terminus (carboxyl terminus), according to the common practice of peptide designation. For PAC1 used in the present invention, the C-terminus may be any of a carboxyl group (-COOH), a carboxylate (-COO⁻), an amide (-CONH₂) or an ester (-COOR).
Here, as R in the ester, a C₁₋₆ alkyl group such as methyl, ethyl, n-propyl, isopropyl and n-butyl, a C₃₋₈ cycloalkyl group such as cyclopentyl and cyclohexyl, a C₆₋₁₂ aryl group such as phenyl and α-naphthyl, a phenyl-C₁₋₂ alkyl group such as benzyl and phenethyl, a C₇₋₁₄ aralkyl group such as an α,-naphthyl-C₁₋₂ alkyl group such as α-naphthylmethyl, a pivaloyloxymethyl group; and the like can be used.
When the PAC1 to be used in the present invention has a carboxyl group (or a carboxylate) in addition to that on the C-terminal, one in which the carboxyl group is amidated or esterified is also included in the protein in the present invention. In this case, as the ester, the above-described C-terminal ester and the like, for example, can be used.
Furthermore, the PAC1 to be used in the present invention also includes a protein wherein the amino group of the N-terminal amino acid residue thereof is protected by a protecting group (e.g., a C₁₋₆ acyl group such as C₁₋₆ alkanoyl such as a formyl group or an acetyl group, and the like), a protein wherein the N-terminal glutamine residue, which is produced by cleavage in vivo, has been converted to pyroglutamic acid, a protein wherein a substituent (e.g., -OH, -SH, an amino group, an imidazole group, an indole group, a guanidino group and the like) on an amino acid side chain in the molecule is protected by an appropriate protecting group (e.g., a C₁₋₆ acyl group such as a C₁₋₆ alkanoyl group such as a formyl group or an acetyl group, and the like), a conjugated protein such as what is called a glycoprotein [e.g., in case of human PAC1, for example, Asn residue shown by amino acid numbers 28, 40, 97, 280 and 355 in the amino acid sequence shown by SEQ ID NO: 2 can be mentioned as potential sugar chain addition sites (based on GPCR database (http://br.expasy.org/uniprot/PACR_HUMAN))], which has a sugar chain bound thereto, and the like.

A partial peptide of PAC1 may be any one, as far as it is a peptide having a partial amino acid sequence of PAC1 described above, and having substantially the same quality of activity as PAC1. Here, "substantially the same quality of activity" is as described above. A measurement of "substantially the same quality of activity" can be performed in the same manner as in the case of PAC1.
Specifically, as a partial peptide of PAC1, for example, one having a partial amino acid sequence of the amino acid sequence shown by SEQ ID NO:2, comprising a region involved in the binding to the ligand PACAP [for example, in the case of human PAC1, for example, the extracellular regions shown by amino acid numbers 1-135, 186-207, 272-289 and 352-365 in the amino acid sequence shown by SEQ ID NO:2 (based on the GPCR database (http://br.expasy.org/uniprot/PACR_HUMAN)) and the like] or a region involved in signal transduction via an interaction with PACAP [for example, a region having an activity to bind to the α subunit (Gα) of trimeric G protein to promote the GDP-GTP exchange reaction thereof (for example, cytoplasmic 3rd loop region) and the like], and the like are used. Although the size of a partial peptide of PAC1 is not particularly limited, examples include those comprising a partial amino acid sequence consisting of 10 or more, preferably 30 or more, more preferably 100 or more, and more preferably 200 or more, amino acid residues. The partial amino acid sequence may be a single continuous partial amino acid sequence, or may be one wherein a plurality of incontinuous partial amino acid sequences are joined.

In addition, for the partial peptide of the PAC1, the C-terminus may be any of a carboxyl group (-COOH), a carboxylate (-COO⁻), an amide (-CONH₂) or an ester (-COOR). Here, as R in the ester, the same as those mentioned for the PAC1 can be mentioned. When a partial peptide of PAC1 has a carboxyl group (or a carboxylate) in addition to that on the C-terminal, one in which the carboxyl group is amidated or esterified is also included in the partial peptide of PAC1. In this case, as the ester, those similar to the C-terminal ester and the like, for example, can be used.
Furthermore, partial peptides of PAC1, like PAC1 as described above, also include those having the amino group of the N-terminal amino acid residue protected by a protecting group; those having the N-terminal glutamine residue pyroglutamated; those having a substituent on a side chain of an amino acid in the molecule protected by an appropriate protecting group, or complex peptides having a sugar chain bound thereto, such as what is called a glycopeptide, and the like.

The PAC1 or partial peptide thereof used in the present invention may be in the form of a salt. For example, salts with physiologically acceptable acids (e.g., inorganic acids, organic acids), bases (e.g., alkali metals) and the like are used; particularly, physiologically acceptable acid addition salts are particularly preferable. Such salts include, for example, salts with inorganic acids (e.g., hydrochloric acid, phosphoric acid, hydrobromic acid, sulfuric acid), or salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

PAC1 can be produced from a cell or tissue of a mammal mentioned above by a method of protein purification known per se. Specifically, PAC1 or a salt thereof can be prepared by homogenizing a tissue or cell of a mammal, removing the cell debris by low speed centrifugation, thereafter precipitating a cell membrane-containing fraction by high speed centrifugation of the supernatant (and, where necessary, purifying the cell membrane fraction by density gradient centrifugation and the like), and subjecting the fraction to a chromatography such as reversed-phase chromatography, ion exchange chromatography, or affinity chromatography, and the like. As described below, in a preferred embodiment of the present invention, PAC1 is purified as a PAC1/detergent complex from the above-described cell membrane-containing fraction using an appropriate detergent.

PAC1 or a partial peptide thereof (hereinafter, in the description of chemical synthesis thereof, these are generically simply referred to as "PAC1" unless otherwise specified) can also be produced by a publicly known method of peptide synthesis.
The method of peptide synthesis may be any of, for example, a solid phase synthesis process and a liquid phase synthesis process. That is, a desired protein can be produced by condensing a partial peptide or amino acids capable of constituting the PAC1 and the remaining portion, and eliminating any protecting group the resultant product may have.
As examples of the commonly known methods of condensation and elimination of the protecting group, the methods described in (1) to (5) below can be mentioned.
(i) M. Bodanszky and M.A. Ondetti, Peptide Synthesis, Interscience Publishers, New York (1966)
(ii) Schroeder and Luebke, The Peptide, Academic Press, New York (1965)
(iii) Nobuo Izumiya, et al.: Peptide Gosei-no-Kiso to Jikken, published by Maruzen Co. (1975);
(iv) Haruaki Yajima and Shunpei Sakakibara: Seikagaku Jikken Koza 1, Tanpakushitsu no Kagaku IV, 205 (1977)
(v) Haruaki Yajima, ed.: Zoku Iyakuhin no Kaihatsu, Vol. 14, Peptide Synthesis, published by Hirokawa Shoten.

PAC1 thus obtained can be purified and isolated by a known purification method. Examples of the purification method include solvent extraction, distillation, column chromatography, liquid chromatography, recrystallization and a combination of these.
When the PAC1 obtained by the above-described method is a free form, the free form can be converted to an appropriate salt by a publicly known method or a method based thereon; conversely, when the PAC1 is obtained in the form of a salt, the salt can be converted to a free form or another salt by a publicly known method or a method based thereon.

For the synthesis of the PAC1, an ordinary commercially available resin for protein synthesis can be used. As examples of such resins, chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenylacetamidomethyl resin, polyacrylamide resin, 4-(2',4'-dimethoxyphenyl-hydroxymethyl)phenoxy resin, 4-(2',4'-dimethoxyphenyl-Fmoc-aminoethyl)phenoxy resin and the like can be mentioned. Using such a resin, an amino acid having an appropriately protected α-amino group and side chain functional group is condensed on the resin in accordance with the sequence of the desired PAC1 according to various methods of condensation known *per se.* At the end of the reaction, the protein and the like are cleaved from the resin, various protecting groups are removed simultaneously, and a reaction to form an intramolecular disulfide bond is carried out in a highly diluted solution to obtain the desired PAC1 or an amide thereof.

For the above-described condensation of protected amino acids, various activation reagents useful for protein synthesis can be used, with preference given to a carbodiimide. As the carbodiimide, DCC, N,N'-diisopropylcarbodiimide, N-ethyl-N'-(3-dimethylaminoprolyl) carbodiimide and the like can be used. For the activation using these carbodiimides, the protected amino acid, along with a racemation-suppressing additive (e.g., HOBt, HOOBt), may be added directly to the resin, or the protected amino acid may be activated in advance as a symmetric acid anhydride, or HOBt ester or HOOBt ester and then added to the resin.

A solvent used for activation of protected amino acids and condensation of protected amino acids with a resin can be appropriately selected from among solvents that are known to be usable for protein condensation reactions. Examples of such useful solvents include acid amides such as N,N-dimethylformamide, N,N-dimethylacetamide, and N-methylpyrrolidone; halogenated hydrocarbons such as methylene chloride and chloroform; alcohols such as trifluoroethanol; sulfoxides such as dimethyl sulfoxide; amines such as pyridine; ethers such as dioxane and tetrahydrofuran; nitriles such as acetonitrile and propionitrile; esters such as methyl acetate and ethyl acetate; suitable mixtures thereof; and the like. Reaction temperature is appropriately selected from the range that is known to be usable in protein binding reactions, and is normally from the range of about -20°C to about 50°C.
An activated amino acid derivative is normally used from 1.5 to 4 times in excess. When the condensation is insufficient as the result of the test using a ninhydrin reaction, sufficient condensation can be carried out by repeating the condensation reaction without elimination of the protecting group. If the condensation is insufficient even though the condensation reaction is repeated, unreacted amino acids can be acetylated by using acetic anhydride or acetylimidazole.

A protecting method and a protecting group of a functional group that should not been involved in the reaction of starting materials, a method of removing the protecting group, a method of activating a functional group involved in the reaction, and the like can be appropriately selected from among publicly known groups or publicly known means.
As the protecting group for the amino group of the starting material, for example, Z, Boc, tertiary pentyloxycarbonyl, isobornyloxycarbonyl, 4-methoxybenzyloxycarbonyl, C1-Z, Br-Z, adamantyloxycarbonyl, trifluoroacetyl, phthaloyl, formyl, 2-nitrophenylsulfenyl, diphenylphosphinothioyl, Fmoc and the like can be used.
The carboxyl group can be protected by, for example, alkyl esterification (e.g., linear, branched or cyclic alkyl esterification with methyl, ethyl, propyl, butyl, tertiary butyl, cyclopentyl, cyclohexyl, cycloheptyl, cyclooctyl, 2-adamantyl, and the like), aralkyl esterification (e.g., benzyl esterification, 4-nitrobenzyl esterification, 4-methoxybenzyl esterification, 4-chlorobenzyl esterification, benzhydryl esterification), phenacyl esterification, benzyloxycarbonyl hydrazidation, tertiary butoxycarbonyl hydrazidation, trityl hydrazidation, and the like.
The hydroxyl group of serine can be protected by, for example, esterification or etherification. As the group suitable for this esterification, for example, lower alkanoyl groups such as an acetyl group, aroyl groups such as a benzoyl group, and groups derived from carbonic acid such as benzyloxycarbonyl group, ethoxycarbonyl group and the like can be used. In addition, as examples of the group suitable for etherification, for example, a benzyl group, a tetrahydropyranyl group, a t-butyl group and the like can be mentioned.
As the protecting group for the phenolic hydroxyl group of tyrosine, for example, Bzl, Cl₂-Bzl, 2-nitrobenzyl, Br-Z, tertiary butyl and the like can be used.
As the protecting group for the imidazole of histidine, for example, Tos, 4-methoxy-2,3,6-trimethylbenzenesulfonyl, DNP, benzyloxymethyl, Bum, Boc, Trt, Fmoc and the like can be used.

As the method of removing (eliminating) a protecting group, catalytic reduction in a hydrogen stream in the presence of a catalyst such as Pd-black or Pd-carbon; acid treatment by means of anhydrous hydrogen fluoride, methanesulfonic acid, trifluoromethane-sulfonic acid, trifluoroacetic acid, or a mixture solution thereof; base treatment by means of diisopropylethylamine, triethylamine, piperidine, piperazine or the like; and reduction with sodium in liquid ammonia, and the like, for example, can be used. The elimination reaction by the above-described acid treatment is generally carried out at a temperature of about -20°C to about 40°C; the acid treatment is efficiently conducted by adding a cation scavenger such as, for example, anisole, phenol, thioanisole, m-cresol, p-cresol, dimethyl sulfide, 1,4-butanedithiol or 1,2-ethanedithiol. Also, a 2,4-dinitrophenyl group used as a protecting group for the imidazole of histidine is removed by thiophenol treatment; a formyl group used as a protecting group for the indole of tryptophan is removed by acid treatment in the presence of 1,2-ethanedithiol, 1,4-butanedithiol, or the like, as well as by alkali treatment with a dilute sodium hydroxide solution, dilute ammonia, or the like.

As the starting material having an activated carboxyl group, for example, a corresponding acid anhydride, an azide, an activated ester [an ester with an alcohol (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, or HOBt)] and the like can be used. As the starting material having an activated amino group, for example, a corresponding phosphoric amide can be used.

In another method of obtaining an amide of PAC1, for example, first, the α-carboxyl group of the carboxy-terminal amino acid is protected by amidation, after which the peptide chain is extended to a desired chain length toward the amino group side, after which a protein (peptide) wherein only the protecting group for the α-amino group at the N-terminus of the peptide chain has been removed and a protein (peptide) wherein only the protecting group for the carboxyl group at the C-terminus has been removed are produced, and these two proteins (peptides) are condensed in a mixed solvent described above. The details of the condensation reaction are as mentioned above. After purification of the protected protein (protected peptide) obtained by condensation, all the protecting group is eliminated by the above-mentioned method to give a desired crude protein (crude peptide). The crude protein (crude peptide) is purified by various known purification means, and the main fraction is lyophilized to give an amide of the desired PAC1.
An ester of the PAC1 can be obtained, for example, by condensing the α-carboxyl group of carboxy terminal amino acid with a desired alcohol to give an amino acid ester, and treating the ester in the same manner as in amide of the above-mentioned PAC1.

The partial peptide of the PAC1 can also be produced by cleaving the PAC1 full-length protein with an appropriate peptidase.

As described below, the PAC1 or partial peptide thereof used as an immunogen in the present invention is preferably prepared as a complex with a detergent, so as to allow the same to retain the native structure thereof (in the case of a partial peptide, a structure in a state allowing the partial peptide to exhibit the same quality of function as the essential physiological function of PAC1). Therefore, a PAC1 or partial peptide thereof synthesized chemically as described above is preferably mixed with an appropriate detergent before immunization of an animal, to prepare a complex whose hydrophobic region is covered with a detergent micelle.

Moreover, a PAC1 can also be produced by culturing a transformant having the nucleic acid encoding same, and separating and purifying a PAC1 from the obtained culture. The nucleic acid that encodes PAC1 or a partial peptide thereof may be DNA or RNA, or a DNA/RNA chimera, and is preferably DNA. In addition, the nucleic acid may be a double-strand, or single-strand. The double-strand may be a double-stranded DNA, a double-stranded RNA, or a DNA:RNA hybrid. In the case of a single strand, it may be a sense strand (i.e., coding strand) or an antisense strand (i.e., non-coding strand).
As the DNA that encodes PAC1 or a partial peptide thereof, genomic DNAs, cDNAs derived from any cell [for example, hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell, fibroblast, fibrocyte, myocyte, adipocyte, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, interstitial cell, or a corresponding precursor cell, stem cell or cancer cell thereof, and the like] of a mammal (for example, human, cattle, monkey, horse, pig, sheep, goat, dog, cat, guinea pig, rat, mouse, rabbit, hamster and the like), or any tissue in which these cells are present [for example, brain or any portion of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gallbladder, bone marrow, adrenal gland, skin, lung, gastrointestinal tract (e.g., large intestine and small intestine), blood vessel, heart, thymus, spleen, submandibular gland, peripheral blood, prostate, testicle, ovary, placenta, uterus, bone, joint, adipose tissue (e.g., brown adipose tissue, white adipose tissue), skeletal muscle and the like], synthetic DNAs and the like can be mentioned.
A genomic DNA and cDNA encoding PAC1 or a partial peptide thereof can also be directly amplified by Polymerase Chain Reaction (hereinafter to be abbreviated as "PCR method") or Reverse Transcriptase-PCR (hereinafter to be abbreviated as "RT-PCR method"), using genomic DNA fraction or total RNA or mRNA fraction prepared from the above-mentioned cell/tissue as a template. Alternatively, genomic DNA or cDNA encoding PAC1 or a partial peptide thereof can also be cloned by colony or plaque hybridization method, PCR method and the like, from the genomic DNA library or cDNA library prepared by inserting, into a suitable vector, a fragment of genomic DNA and total RNA or mRNA prepared from the above-mentioned cell/tissue. The vector to be used for the library may be any of bacteriophage, plasmid, cosmid, phagemid and the like.

An example of the DNA that encodes the PAC1 includes, a DNA that has a base sequence shown by SEQ ID NO: 1, a DNA that has a base sequence hybridizing to a base sequence shown by SEQ ID NO: 1 under stringent conditions and encodes a protein having substantially the same quality of activity as the aforementioned PAC1 (that is, ligand binding activity, the signal transduction activity, etc.) or the like.
Useful DNA capable of hybridizing with the base sequence shown by SEQ ID NO:1 under stringent conditions include, for example, a DNA comprising a base sequence having a homology of about 60% or more, preferably about 70% or more, more preferably about 80% or more, particularly preferably about 90% or more, to the base sequence shown by SEQ ID NO:1.
Homology of the base sequences in the present specification can be calculated under the following conditions (an expectation value = 10; gaps are allowed; filtering = ON; match score = 1; mismatch score = -3) using a homology scoring algorithm NCBI BLAST (National Center for Biotechnology Information Basic Local Alignment Search Tool). As other algorithm with which to determine the homology of the base sequence, the homology calculation algorithm of the above-mentioned amino acid sequence can be preferably used in the same manner.

The hybridization can be performed by a method known per se or a method analogous thereto, for example, a method described in Molecular Cloning, 2nd ed. (J. Sambrook et al., Cold Spring Harbor Lab. Press, 1989) and the like. A commercially available library can also be used according to the instructions of the attached manufacturer's protocol. Preferably, the hybridization can be carried out under stringent conditions.
As examples of high-stringent conditions, a hybridization reaction in 6×SSC (sodium chloride/sodium citrate) at 45°C followed by 1 or more times of washing in 0.2×SSC/0.1% SDS at 65°C and the like can be mentioned. Those skilled in the art can easily regulate the conditions to obtain a desired stringency by appropriately changing the salt concentration of hybridization solution, hybridization reaction temperature, probe concentration, probe length, number of mismatches, hybridization reaction time, salt concentration of washing solution, washing temperature, and the like.

The DNA that encodes PAC1 is preferably a DNA comprising a base sequence that encodes the human PAC1 protein represented by the base sequence shown by SEQ ID NO:1 (GenBank accession number: NM_001118), or an orthologue thereof in another mammal (for example, mouse and rat orthologues registered under accession numbers NM_007407 and NM_133511, respectively, in the GenBank and the like).

The DNA that encodes a partial peptide of PAC1 may be any one, as far as it comprises a base sequence that encodes a peptide having the same or substantially the same amino acid sequence as a portion of the amino acid sequence shown by SEQ ID NO:2, and encodes a peptide having a bioactivity equivalent to that of PAC1. Specifically, as the DNA that encodes a partial peptide of PAC1, for example, a DNA that has (1) a partial base sequence of the base sequence shown by SEQ ID NO:1 or (2) a base sequence that hybridizes with a DNA having the base sequence shown by SEQ ID NO:1 under stringent conditions, and that encodes a peptide having substantially the same quality of activity (e.g., ligand binding activity, signal transduction activity and the like) as the foregoing PAC1, and the like are used.

The DNA that encodes the PAC1 or a partial peptide thereof can be cloned by amplifying it by the PCR method using a synthetic DNA primer having a portion of the base sequence that encodes the PAC1 or a partial peptide thereof, or by hybridizing DNA incorporated in an appropriate expression vector to a labeled DNA fragment or synthetic DNA that encodes a portion or the entire region of the PAC1 protein. The hybridization can be performed by, for example, a method described in Molecular Cloning, 2nd ed. (ibid.) and the like. A commercially available library can also be used according to the instructions of the manufacturer's protocol attached thereto.

The base sequence of the DNA can be converted according to a method known per se, such as the ODA-LA PCR method, the Gapped duplex method, or the Kunkel method, or a method based thereon, using a commonly known kit, for example, Mutan^{™}-super Express Km (TAKARA SHUZO CO. LTD.), Mutan^{™}-K (TAKARA SHUZO CO. LTD.) and the like.

The cloned DNA can be used as is, or after digestion with a restriction endonuclease or addition of a linker as desired, depending on the purpose of its use. The DNA may have the translation initiation codon ATG at the 5' end thereof, and the translation stop codon TAA, TGA or TAG at the 3' end thereof. These translation initiation codons and translation stop codons can be added by using a suitable synthetic DNA adaptor.

An expression vector containing a DNA encoding PAC1 or a partial peptide thereof can be produced, for example, by cleaving out an object DNA fragment from the DNA encoding PAC1 and connecting the DNA fragment with the downstream of a promoter in a suitable expression vector.
As expression vectors, plasmids derived from *Escherichia* coli (e.g., pBR322, pBR325, pUC12, pUC13); plasmids derived from *Bacillus subtilis* (e.g., pUB110, pTP5, pC194); plasmids derived from yeast (e.g., pSH19, pSH15); bacteriophages such as λ phage; insect cell expression plasmids (e.g., pFast-Bac); animal cell expression plasmids (e.g., pA1-11, pXT1, pRc/CMV, pRc/RSV, pcDNAI/Neo); insect virus vectors such as baculovirus (e.g., BmNPV, AcNPV); animal virus vectors such as retrovirus, vaccinia virus, and adenovirus, and the like are used.
The promoter may be any promoter appropriate for the host used to express the gene.
For example, when the host is an animal cell, the SRα promoter, the SV40 promoter, the LTR promoter, the CMV (cytomegalovirus) promoter, the RSV (Rous sarcoma virus) promoter, the MoMuLV (Moloney mouse leukemia virus) LTR, the HSV-TK (herpes simplex virus thymidine kinase) promoter and the like are used. Particularly, the CMV promoter, the SRα promoter and the like are preferable.
When the host is a bacterium of the genus Escherichia, the trp promoter, the lac promoter, the recA promoter, the λP_{L} promoter, the lpp promoter, the T7 promoter and the like are preferred.
When the host is a bacterium of the genus Bacillus, the SPO1 promoter, the SPO2 promoter, the penP promoter and the like are preferred.
When the host is yeast, the PHO5 promoter, the PGK promoter, the GAP promoter, the ADH promoter and the like are preferred.
When the host is an insect cell, the polyhedrin promoter, the P10 promoter and the like are preferred.

Useful expression vectors include, in addition to the above, expression vectors that optionally comprise an enhancer, a splicing signal, a polyA addition signal, a selection marker, an SV40 replication origin (hereinafter also abbreviated as SV40ori), and the like. As examples of the selection markers, the dihydrofolate reductase (hereinafter also abbreviated as dhfr) gene [methotrexate (MTX) resistance], the ampicillin resistance gene (hereinafter also abbreviated as Amp^{r}), the neomycin resistance gene (hereinafter also abbreviated as Neo^{r}, G418 resistance), and the like can be mentioned. In particular, when a dhfr gene-defective Chinese hamster ovary cell is used and the dhfr gene is used as the selection marker, a target gene can also be selected using a thymidine-free medium.
In addition, where necessary, a base sequence (single codon) encoding a signal sequence suitable for the host may be added to the 5' end side of DNA encoding PAC1 or a partial peptide thereof (or substituted by a native signal codon). Useful signal sequences include a PhoA signal sequence, an OmpA signal sequence and the like when the host is a bacterium of the genus Escherichia; an α-amylase signal sequence, a subtilisin signal sequence and the like when the host is a bacterium of the genus Bacillus; an MFα signal sequence, an SUC2 signal sequence and the like when the host is yeast; and an insulin signal sequence, an α-interferon signal sequence, an antibody molecule signal sequence and the like when the host is an animal cell.

By transforming a host with the above-described expression vector comprising a DNA that encodes PAC1 or a partial peptide thereof, and culturing the transformant obtained, PAC1 or a partial peptide thereof can be produced.
As useful examples of the host, a bacterium of the genus Escherichia, a bacterium of the genus Bacillus, yeast, an insect cell, an insect, an animal cell, and the like can be mentioned.
Useful bacteria of the genus Escherichia include, for example, Escherichia coli K12•DH1, Escherichia coli JM103, Escherichia coli JA221, Escherichia coli HB101, Escherichia coli C600 and the like.
Useful bacteria of the genus Bacillus include, for example, Bacillus subtilis MI114, Bacillus subtilis 207-21 and the like.
As useful examples of the yeast, Saccharomyces cerevisiae AH22, AH22R⁻, NA87-11A, DKD-5D and 20B-12, Schizosaccharomyces pombe NCYC1913 and NCYC2036, Pichia pastoris KM71 and the like can be mentioned.

As useful examples of the insect cell, Spodoptera frugiperda cell (Sf cell), MG1 cell derived from the mid-intestine of Trichoplusia ni, High Five^{™} cell derived from an egg of Trichoplusia ni, cell derived from Mamestra brassicae, cell derived from Estigmena acrea, and the like can be mentioned when the virus is AcNPV. When the virus is BmNPV, Bombyx mori N cell (BmN cell) and the like can be used. As useful examples of the Sf cell, Sf9 cell (ATCC CRL1711), Sf21 cell, and the like can be mentioned.
As useful examples of the insect, a larva of Bombyx mori and the like can be mentioned.

Useful animal cells include, for example, cells such as COS-7, Vero, CHO, CHO(dhfr⁻), CHO-K1, L, AtT-20, GH3, FL, HEK293, NIH3T3, Balb3T3, FM3A, L929, SP2/0, P3U1, B16, and P388.

Transformation can be performed according to the choice of host by a commonly known method.
A bacterium of the genus *Escherichia* can be transformed, for example, in accordance with a method described in Proc. Natl. Acad. Sci. USA, Vol.69, 2110 (1972), Gene, Vol.17, 107 (1982) and the like.
A bacterium of the genus *Bacillus* can be transformed, for example, according to a method described in Molecular & General Genetics, Vol.168, 111 (1979) and the like.
Yeast can be transformed, for example, in accordance with a method described in Methods in Enzymology, Vol.194, 182-187 (1991), Proc. Natl. Acad. Sci. USA, Vol.75, 1929 (1978) and the like.
An insect cell or insect can be transformed, for example, according to a method described in Bio/Technology, 6, 47-55 (1988) and the like.
An animal cell can be transformed, for example, in accordance with a method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, Vol.52, 456 (1973).

Transformation can be performed according to the choice of host by a commonly known method.
When a transformant whose host is a bacterium of the genus *Escherichia* or a bacterium of the genus *Bacillus* is cultured, the culture medium used is preferably a liquid medium, in which a carbon source, a nitrogen source, an inorganic substance and others necessary for the growth of the transformant are preferably contained. Here, as examples of the carbon source, glucose, dextrin, soluble starch, sucrose and the like can be mentioned; as examples of the nitrogen source, inorganic or organic substances such as an ammonium salt, a nitrate salt, corn steep liquor, peptone, casein, meat extract, soybean cake, and potato extract can be mentioned; as examples of the inorganic substance, calcium chloride, sodium dihydrogen phosphate, magnesium chloride and the like can be mentioned. In addition, yeast extract, vitamins, a growth promoting factor and the like may be added to the medium. The pH of the medium is preferably about 5 to about 8.
As an example of the medium used to culture a bacterium of the genus *Escherichia,* an M9 medium comprising glucose and casamino acid is preferable. As required, in order to increase promoter efficiency, a chemical agent, for example, 3β-indolylacrylic acid, may be added to the medium.
When the host is a bacterium of the genus *Escherichia,* cultivation is normally performed at about 15 to about 43°C for about 3 to about 24 hours, and the culture may be aerated or agitated as necessary.
When the host is a bacterium of the genus *Bacillus*, cultivation is normally performed at about 30 to about 40°C for about 6 to about 24 hours, and the culture may be aerated or agitated as necessary.
When a transformant whose host is yeast is cultured, as examples of the medium, Burkholder's minimal medium and an SD medium supplemented with 0.5% casamino acid can be mentioned. The pH of the medium is preferably about 5 to about 8.
Cultivation is normally performed at about 20°C to about 35°C for about 24 to about 72 hours, and the culture may be aerated or agitated as necessary.
When a transformant whose host is an insect cell or insect is cultured, as the medium, Grace's Insect Medium supplemented with inactivated 10% bovine serum and other additives as appropriate and the like are used. The pH of the medium is preferably about 6.2 to about 6.4. Cultivation is normally performed at about 27°C for about 3 to about 5 days, and the culture may be aerated or agitated as necessary.
Useful medium for cultivating a transformant whose host is an animal cell include, for example, minimum essential medium (MEM) supplemented with about 5 to 20% fetal bovine serum, Dulbecco's modified Eagle medium (DMEM), RPMI 1640 medium, 199 medium and the like. The medium's pH is preferably about 6 to about 8. Cultivation is normally performed at about 30 to about 40°C for about 15 to about 60 hours, and the culture may be aerated or agitated as necessary.
Thus, the PAC1 or a partial peptide thereof can be produced in or outside the cells of the transformant.

The PAC1 or a partial peptide thereof can be separated and purified from the culture obtained by cultivating the aforementioned transformant according to a method known *per se.*
For example, when PAC1 or a partial peptide thereof is extracted from a membrane fraction, useful methods include a method wherein cell bodies or cells collected from a culture by a publicly known method are suspended in an appropriate buffer solution, and the cell bodies or cells are disrupted by the homogenizer method, sonication, lysozyme and/or freeze-thawing and the like, after which the cell debris is precipitated and removed via low-speed centrifugation, the supernatant is centrifuged at high speed to precipitate a cell membrane-containing fraction (if necessary, the cell membrane fraction is purified by density gradient centrifugation and the like) and the like.
Isolation and purification of the PAC1 or a partial peptide thereof contained in the thus-obtained membrane fraction can be conducted according to a method know *per se.* Useful methods include methods based on solubility, such as salting-out and solvent precipitation; methods based mainly on differences in molecular weight, such as dialysis, ultrafiltration, gel filtration, and SDS-polyacrylamide gel electrophoresis; methods based on differences in electric charge, such as ion exchange chromatography; methods based on specific affinity, such as affinity chromatography; methods based on differences in hydrophobicity, such as reverse phase high performance liquid chromatography; methods based on differences in isoelectric point, such as isoelectric focusing; and the like. These methods can be combined as appropriate.

When the thus-obtained protein or peptide is a free form, the free form can be converted to a salt by a method known per se or a method based thereon; when the protein or the peptide is obtained as a salt, the salt can be converted to a free form or another salt by a method known *per se* or a method based thereon.
Note that the PAC1 or a partial peptide thereof produced by the transformant can be optionally modified or partially deprived of a polypeptide by allowing an appropriate protein-modifying enzyme to act thereon before the purification or after the purification. As the protein-modifying enzyme used, for example, trypsin, chymotrypsin, arginyl endopeptidase, protein kinase, glycosidase and the like are used.

As described below, in a preferred embodiment of the present invention, PAC1 is purified as a PAC1/detergent complex from the above-described membrane fraction using an appropriate detergent.

Furthermore, the PAC1 or a partial peptide thereof can also be synthesized in vitro using a cell-free protein translation system that comprises a rabbit reticulocyte lysate, wheat germ lysate, *Escherichia coli* lysate and the like, with RNA corresponding to the above-described DNA that encodes the above-mentioned PAC1 or a partial peptide thereof as the template. Alternatively, PAC1 or a partial peptide thereof can be synthesized using a cell-free transcription/translation system containing RNA polymerase, with the DNA that encodes PAC1 or a partial peptide thereof as the template. As the cell-free protein transcription/translation system, commercially available ones can be used, a method known *per se,* and specifically, an *Escherichia* coli extract can also be prepared by the method described in Pratt J. M. et al., Transcription and Translation, Hames B. D. & Higgins S. J. eds., IRL Press, Oxford, 179-209 (1984) and the like. As the commercially available cell lysates, as those derived from *Escherichia* coli, *E. coli* S30 extract system (manufactured by Promega), RTS 500 Rapid Translation System (manufactured by Roche) and the like can be mentioned, as those derived from rabbit reticulocyte, Rabbit Reticulocyte Lysate System (manufactured by Promega) and the like can be mentioned, and as those derived from wheat germ, PROTEIOS^{™} (manufactured by TOYOBO) and the like can be mentioned. Of these, ones using a wheat germ lysate are preferable. As the production method of wheat germ lysate, for example, the methods described in Johnston F.B. et al., Nature, 179: 160-161 (1957) or Erickson A.H. et al., Meth. Enzymol., 96: 38-50 (1996) and the like can be used.
As the system or apparatus for protein synthesis, batch method (Pratt, J.M. et al. (1984), mentioned above), continuous cell-free protein synthesis system (Spirin A.S. et al., Science, 242: 1162-1164 (1988)) wherein amino acid, energy source and the like are continuously supplied to the reaction system, dialysis (Kikawa et al., The 21st Annual Meeting of the Molecule Biology Society of Japan, WID6), or overlay method (manual of PROTEIOS^{™} Wheat germ cell-free protein synthesis core kit: manufactured by TOYOBO) and the like can be mentioned. Moreover, a method (JP-A-2000-333673) wherein template RNA, amino acid, energy source and the like are supplied to a synthesis reaction system as necessary and a synthesized substance and decomposed product are discharged as necessary, and the like can be used.

As described below, the PAC1 or a partial peptide thereof used as an immunogen in the present invention is preferably prepared as a complex with a detergent, so as to allow the same to retain the native structure thereof (in the case of a partial peptide, a structure in a state allowing the partial peptide to exhibit the same quality of function as the essential physiological function of PAC1). Therefore, PAC1 or a partial peptide thereof synthesized using a cell-free system as described above is preferably mixed with an appropriate detergent before immunization of an animal, to prepare a complex whose hydrophobic region is covered with a detergent micelle.

A partial peptide of PAC1 permits direct use for immunization in an insolubilized form, as long as it has immunogenicity; when an antigen of low molecular weight (for example, molecular weight about 3,000 or less) having only one to several antigenic determinants in the molecule thereof is used, it can be used for immunization in the form of a complex bound or adsorbed to a suitable carrier because these antigens are normally hapten molecules of low immunogenicity. As the carrier, a naturally occurring or synthetic polymer can be used. As examples of the naturally occurring polymer, serum albumin of a mammal such as bovine, rabbit, or human, thyroglobulin of a mammal such as bovine or rabbit, ovalbumin of chicken, hemoglobin of a mammal such as bovine, rabbit, human, or sheep, keyhole limpet hemocyanin (KLH) and the like can be used. As examples of the synthetic polymer, various latexes of polymers or copolymers of polyamino acids, polystyrenes, polyacryls, polyvinyls, polypropylenes and the like, and the like can be mentioned.

Regarding the mixing ratio of the carrier and hapten, any combination in any ratio can be bound or adsorbed, as long as an antibody against the antigen bound or adsorbed to the carrier is produced efficiently; usually, one wherein the above-described naturally occurring or synthetic polymer carrier in common use in preparing an antibody against hapten is bound or adsorbed in a ratio by weight of 0.1 to 100 to 1 of hapten can be used.

In addition, various condensing agents can be used for coupling the hapten and carrier. For example, diazonium compounds such as bisdiazotized benzidine, which crosslink tyrosine, histidine, and tryptophan; dialdehyde compounds such as glutaraldehyde, which crosslink amino groups together; diisocyanate compounds such as toluene-2,4-diisocyanate; dimaleimide compounds such as N,N'-o-phenylenedimaleimide, which crosslink thiol groups together; maleimide activated ester compounds, which crosslink amino groups and thiol groups; carbodiimide compounds, which crosslink amino groups and carboxyl groups; and the like can be used advantageously. When amino groups are crosslinked together, it is also possible to react one amino group with an activated ester reagent having a dithiopyridyl group (e.g., N-succinimidyl 3-(2-pyridyldithio)propionate (SPDP) and the like), followed by reduction, to introduce the thiol group, and to introduce a maleimide group into the other amino group using a maleimide activated ester reagent, followed by a reaction of both.

Alternatively, a warm-blooded animal or insect cell that expresses PAC1, as is, or a cell membrane-containing fraction thereof, can also be used directly as an antigen. As the warm-blooded animal or insect cell, a cell that endogenously produces PAC1, a cell transformed with a DNA that encodes PAC1 and the like can be used, but because endogenous PAC1 is expressed at low levels, it is more preferable to use a transformant cell having exogenous PAC1 expressed forcibly in a large amount. The membrane fraction can also be fused with liposome using various publicly known methods, and used as a proteoliposome. For example, a method wherein a membrane fraction and liposome are mixed in an appropriate ratio, after which freeze-thawing is repeated, a method wherein an aqueous solution containing the membrane fraction is placed on a film prepared from a lipid mixture, after which the membrane protein is transferred to a lipid bilayer by the hydration method and the like can be used for this purpose. Preference is given to freeze-thawing. The proteoliposome obtained can be subjected to sizing by ultrasound treatment, the homogenizer method, the filtration method (extruder method) and the like. These cells or membrane fraction can be injected to an animal for immunization in a state suspended in a medium (e.g., RPMI1640) or a buffer solution (e.g., Hanks' Balanced Salt Solution). The method of immunization may be any method, as far as it allows promotion of antibody production; intravenous injection, intraperitoneal injection, intramuscular injection, subcutaneous injection and the like are preferably used.

In a preferred embodiment of the present invention, the PAC1 or partial peptide thereof used as an immunogen is provided as a complex with an appropriate detergent.
To isolate and purify PAC1 or a partial peptide thereof (hereinafter, in the description of preparing a complex with a detergent, both are together abbreviated as "PAC1") while retaining the native structure thereof, solubilization with a detergent is required. To solubilize a membrane protein without denaturing the same, a series of non-ionic or amphoteric ionic detergents called mild detergents are used. To efficiently obtain an antibody capable of recognizing a PAC1 having a native structure, it is necessary to use a PAC1/detergent complex with excellent antigen presenting capacity as an immunogen, but this is significantly influenced by micelle size. The present inventors found, out of various detergents of different sizes, sucrose monoalkylates having an alkyl chain with 8 to 12 carbon atoms, preferably β-D-fructopyranosyl-α-glucopyranoside monododecanoate (SM1200), or alkyl-β-D-maltosides with 8 to 12 carbon atoms, preferably dodecyl-β-D-maltoside (DDM), and the like, as being capable of providing a PAC1/detergent complex with excellent antigen presenting capacity. For example, by using the detergent in combination with an amphiphathic substance such as cholesterol hemisuccinate (CHS), the antigen presenting capacity of the PAC1/detergent complex can be further improved. Although the blending ratio of the detergent and the amphiphathic substance is not particularly limited, it is preferable that, for example, when using a mixed miscelle of SM1200 and CHS, the blending ratio be 10:1 to 10:3. The concentration of the detergent used as a solubilizing agent is normally in the range from about 0.5 to about 5% (w/w).

As PAC1, a cell membrane-containing fraction of a warm-blooded animal or insect cell that expresses the foregoing PAC1 (preferably, a transformant cell having an exogenous PAC1 expressed forcibly therein) is preferably used. A solution of the above-described detergent (containing a mixed micelle with an amphiphathic substance) is added thereto to obtain a protein concentration of about 1 to about 5 mg/mL, and the fraction is incubated at about 4 to about 20°C for about 0.5 to about 24 hours to solubilize the membrane protein. Purification of PAC1 can be performed by, for example, bringing the above-described reaction liquid in contact with a water-insoluble carrier (for example, glass beads, resin, gel and the like) with PACAP immobilized thereon, and capturing the PAC1/detergent complex on the carrier on the basis of PACAP-PAC1 affinity. Alternatively, when a recombinant PAC1 is used, a tag sequence (e.g., His-tag, GST-tag, Myc-tag, MBP-tag and the like) may be joined to the N-terminus or C-terminus thereof. To the site where the PAC1 and the tag are joined, an appropriate peptidase recognition site may be added.

### (2) Preparation of monoclonal antibody

### (a) Preparation of monoclonal antibody-producing cells

The immunogen prepared as mentioned above is administered as is, or along with a carrier or a diluent, to a warm-blooded animal at a site enabling antibody production by the methods such as intraperitoneal injection, intravenous injection, subcutaneous injection, intradermal injection and the like. In order to increase antibody productivity upon the administration, Freund's complete adjuvant or Freund's incomplete adjuvant may be administered. Dosing is normally performed about 2 to 10 times in total every 1 to 6 weeks. As examples of the warm-blooded animal to be used, monkeys, rabbits, dogs, guinea pigs, mice, rats, sheep, goats, donkeys and chickens can be mentioned. Although it is preferable to use a mammal of the same species as the recipient in order to avoid the problem of anti-Ig antibody production, mice and rats are generally preferably used for generating a monoclonal antibody.

Because artificial immunization to humans is ethically difficult, it is preferable, when the antibody of the present invention targets a human for administration, (i) to obtain a human antibody by immunizing a human antibody-producing animal (e.g., mouse) produced according to a method described below, (ii) to produce a chimeric antibody, humanized antibody or fully human antibody according to a method described below, or (iii) to obtain a human antibody using in combination the *in* vitro immunization method and cell immortalization with virus, human-human (or -mouse) hybridoma production technique, phage display method and the like. Note that the *in vitro* immunization method can also be used preferably as a method for obtaining an antibody against an antigen that is unstable and difficult to prepare in large amounts for the purpose of preparing a non-human animal-derived antibody, because there is the possibility of obtaining an antibody against an antigen for which antibody production is suppressed by ordinary immunization, because it is possible to obtain an antibody with an amount of antigen on the nanogram to microgram order, because immunization completes in several days, and for other reasons.

As the animal cells used in the *in vitro* immunization method, lymphocytes, preferably B-lymphocytes and the like, isolated from peripheral blood, spleen, lymph node and the like of a human and the above-described warm-blooded animals (preferably mouse or rat) can be mentioned. For example, in the case of mouse or rat cells, the spleen is extirpated from an about 4- to 12-week-old animal, and splenocytes are separated and rinsed with a appropriate medium [e.g., Dulbecco's modified Eagle medium (DMEM), RPMI1640 medium, Ham's F12 medium and the like], after which the splenocytes are suspended in an antigen-containing medium supplemented with fetal calf serum (FCS; about 5 to 20%) and cultured using a CO₂ incubator and the like for about 4 to 10 days. Examples of the antigen concentration include, but are not limited to, 0.05 to 5 µg. It is preferable to prepare a culture supernatant of thymocytes of an animal of the same strain (preferably at about 1 to 2 weeks of age) according to a conventional method, and to add the supernatant to the medium.

Because it is difficult to obtain a thymocyte culture supernatant in *in vitro* immunization of human cells, it is preferable to perform immunization by adding, to the medium, several kinds of cytokines such as IL-2, IL-4, IL-5, and IL-6 and the like, and if necessary, an adjuvant substance (e.g., muramyldipeptide and the like) along with the antigen.

In preparing a monoclonal antibody, it is possible to establish an antibody-producing hybridoma by selecting an individual or cell population showing an elevated antibody titer from among antigen-immunized warm-blooded animals (e.g., mice, rats) or animal cells (e.g., human, mouse, rat), respectively; collecting spleens or lymph nodes at 2 to 5 days after the final immunization or collecting the cells after 4 to 10 days of cultivation after *in vitro* immunization to isolate antibody-producing cells; and fusing the isolated cells with myeloma cells. A measurement of serum antibody titer can be performed by, for example, reacting a labeled antigen and an antiserum, and thereafter determining the activity of the label bound to the antibody.

Although the myeloma cells are not subject to limitation, as long as they are capable of producing a hybridoma that secretes a large amount of antibody, those that do not produce or secrete the antibody *per se* are preferable, with greater preference given to those of high cell fusion efficiency. To facilitate hybridoma selection, it is preferable to use a cell line that is susceptible to HAT (hypoxanthine, aminopterin, thymidine). As examples of the mouse myeloma cells, NS-1, P3U1, SP2/0, AP-1 and the like can be mentioned; as examples of the rat myeloma cells, R210.RCY3, Y3-Ag 1.2.3 and the like can be mentioned; as examples of the human myeloma cells, SKO-007, GM 1500-6TG-2, LICR-LON-HMy2, UC729-6 and the like can be mentioned.

Fusion operation can be performed according to a known method, for example, the method of Koehler and Milstein [Nature, 256, 495 (1975)]. As a fusion promoter, polyethylene glycol (PEG), Sendai virus and the like can be mentioned, and PEG and the like are preferably used. Although the molecular weight of PEG is not subject to limitation, PEG1000 to PEG6000, which are of low toxicity and relatively low viscosity, are preferable. As examples of the PEG concentration, about 10 to 80%, preferably about 30 to 50%, can be mentioned. As the solution for diluting PEG, various buffers such as serum-free medium (e.g., RPMI1640), complete medium comprising about 5 to 20% serum, phosphate buffered saline (PBS), and Tris buffer can be used. DMSO (e.g., about 10 to 20%) can also be added as desired. As examples of the pH of the fusion solution, about 4 to 10, preferably about 6 to 8 can be mentioned.

The ratio by number of antibody-producing cells (splenocytes) and myeloma cells is preferably about 1:1 to 20:1, and the cell fusion can be efficiently performed by incubation normally at 20 to 40°C, preferably at 30 to 37°C, normally for 1 to 10 minutes.

An antibody-producing cell line can also be obtained by infecting antibody-producing cells with a virus capable of transforming lymphocytes to immortalize the cells. As such viruses, for example, Epstein-Barr (EB) virus and the like can be mentioned. Although the majority of persons have immunity because they have ever been infected with this virus in an asymptomatic infection of infectious mononucleosis, virion is also produced when the ordinary EB virus is used; therefore, appropriate purification must be performed. As an EB system free from the possibility of viral contamination, it is also preferable to use a recombinant EB virus that retains the capability of immortalizing B lymphocytes but lacks the capability of replicating virion (for example, deficiency of the switch gene for transition from latent infection state to lytic infection state and the like).

Because marmoset-derived B95-8 cells secrete EB virus, B lymphocytes can be easily transformed by using a culture supernatant thereof. An antibody-producing B cell line can be obtained by, for example, culturing these cells using a medium supplemented with serum and penicillin/streptomycin (P/S) (e.g., RPMI1640) or a serum-free medium supplemented with a cell growth factor, thereafter separating the culture supernatant by filtration or centrifugation and the like, suspending therein antibody-producing B lymphocytes at a suitable concentration (e.g., about 10⁷ cells/mL), and incubating the suspension normally at 20 to 40°C, preferably at 30 to 37°C, normally for about 0.5 to 2 hours. When human antibody-producing cells are provided as mixed lymphocytes, it is preferable to previously remove T lymphocytes by allowing them to form an E rosette with, for example, sheep erythrocytes and the like, to increase transformation frequency of EB virus, because the majority of persons have T lymphocytes which exhibit cytotoxicity to cells infected with EB virus. It is also possible to select lymphocytes specific for the target antigen by mixing sheep erythrocytes, previously bound with a soluble antigen, with antibody-producing B lymphocytes, and separating the rosette using a density gradient of percoll and the like. Furthermore, because antigen-specific B lymphocytes are capped by adding the antigen in large excess so that they no longer present IgG to the surface, mixing with sheep erythrocytes bound with anti-IgG antibody results in the formation of rosette only by antigen-nonspecific B lymphocytes. Therefore, by collecting a layer of cells that don't form rosette from this mixture using a density gradient of percoll and the like, it is possible to select antigen-specific B lymphocytes.

Human antibody-secreting cells having acquired the capability of proliferating indefinitely by the transformation can be back fused with mouse or human myeloma cells in order to stably sustain the antibody-secreting ability. As the myeloma cells, the same as those described above can be used.

Hybridoma screening and breeding are normally performed using a medium for animal cells (e.g., RPMI1640) containing 5 to 20% FCS or a serum-free medium supplemented with cell growth factors, with the addition of HAT (hypoxanthine, aminopterin, thymidine). As examples of the concentrations of hypoxanthine, aminopterin and thymidine, about 0.1 mM, about 0.4 µM and about 0.016 mM and the like, respectively, can be mentioned. For selecting a human-mouse hybridoma, ouabain resistance can be used. Because human cell lines are more susceptible to ouabain than mouse cell lines, it is possible to eliminate unfused human cells by adding ouabain at about 10⁻⁷ to 10⁻³ M to the medium.

In selecting a hybridoma, it is preferable to use feeder cells or culture supernatants of certain cells. As the feeder cells, an allogenic cell species having a lifetime limited so that it dies after helping the emergence of hybridoma, cells capable of producing large amounts of a growth factor useful for the emergence of hybridoma with their proliferation potency reduced by irradiation and the like, and the like are used. For example, as the mouse feeder cells, splenocytes, macrophage, blood, thymocytes and the like can be mentioned; as the human feeder cells, peripheral blood mononuclear cells and the like can be mentioned. As examples of the cell culture supernatant, primary culture supernatants of the above-described various cells and culture supernatants of various established cell lines can be mentioned.

Moreover, a hybridoma can also be selected by reacting a fluorescein-labeled antigen with fusion cells, and thereafter separating the cells that bind to the antigen using a fluorescence-activated cell sorter (FACS). In this case, efforts for cloning can be lessened significantly because a hybridoma that produces an antibody against the target antigen can be directly selected.

For cloning a hybridoma that produces a monoclonal antibody against the target antigen, various methods can be used.

It is preferable to remove aminopterin as soon as possible because it inhibits many cell functions. In the case of mice and rats, aminopterin can be removed 2 weeks after fusion and beyond because most myeloma cells die within 10 to 14 days. However, a human hybridoma is normally maintained in a medium supplemented with aminopterin for about 4 to 6 weeks after fusion. It is desirable that hypoxanthine and thymidine be removed more than one week after the removal of aminopterin. That is, in the case of mouse cells, for example, a complete medium (e.g., RPMI1640 supplemented with 10% FCS) supplemented with hypoxanthine and thymidine (HT) is added or exchanged 7 to 10 days after fusion. About 8 to 14 days after fusion, visible clones emerge. Provided that the diameter of clone has reached about 1 mm, the amount of antibody in the culture supernatant can be measured.

A measurement of the amount of antibody can be performed by, for example, a method comprising adding the hybridoma culture supernatant to a solid phase (e.g., microplate) to which the target antigen or a derivative thereof or partial peptide thereof (including the partial amino acid sequence used as the epitope) is adsorbed directly or with a carrier, subsequently adding an anti-immunoglobulin (IgG) antibody (an antibody against IgG derived from an animal of the same species as the animal from which the original antibody-producing cells are derived is used) or protein A, which had been labeled with a radioactive substance (e.g., ¹²⁵I, ¹³¹I, ³H, ¹⁴C), enzyme (e.g., β-galactosidase, β-glucosidase, alkaline phosphatase, peroxidase, malate dehydrogenase), fluorescent substance (e.g., fluorescamine, fluorescein isothiocyanate), luminescent substance (e.g., luminol, luminol derivative, luciferin, lucigenin) and the like, and detecting the antibody against the target antigen (epitope) bound to the solid phase, a method comprising adding the hybridoma culture supernatant to a solid phase to which an anti-IgG antibody or protein A is adsorbed, adding the target antigen, a derivative thereof, or a partial peptide thereof labeled with the same labeling reagent as described above, and detecting the antibody against the target antigen (epitope) bound to the solid phase and the like.

Although limiting dilution is normally used as the cloning method, cloning using soft agar and cloning using FACS (described above) are also possible. Cloning by limiting dilution can be performed by, for example, the following procedures, which, however, are not to be construed as limiting.

The amount of antibody is measured as described above, and positive wells are selected. Selected suitable feeder cells are previously added to a 96-well plate. Cells are collected from the antibody-positive wells and suspended in complete medium (e.g., RMPI1640 supplemented with 10% FCS and P/S) to obtain a density of 30 cells/mL; 0.1 mL (3 cells/well) of this suspension is added to the well plate with feeder cells added thereto; a portion of the remaining cell suspension is diluted to 10 cells/mL and sown to other wells (1 cell/well)in the same way; the still remaining cell suspension is diluted to 3 cells/mL and sown to other wells (0.3 cells/well). The cells are cultured for about 2 to 3 weeks until a visible clone appears, when the amount of antibody is measured to select positive wells, and the selected cells are recloned in the same way. In the case of human cells, cloning is relatively difficult, so that a plate in which cells are seeded at 10 cells/well is also prepared. Although a monoclonal antibody-producing hybridoma can be obtained normally by two times of subcloning, it is desirable to repeat recloning regularly for several more months to confirm the stability thereof.

Hybridomas can be cultured *in vitro* or *in vivo*.
As a method of *in vitro* culture, a method comprising gradually scaling up a monoclonal antibody-producing hybridoma obtained as described above, from a well plate, while keeping the cell density at, for example, about 10⁵ to 10⁶ cells/mL, and gradually lowering the FCS concentration, can be mentioned.
As a method of *in vivo* culture, for example, a method comprising an intraperitoneal injection of a mineral oil to a mouse (a mouse that is histocompatible with the parent strain of the hybridoma) to induce plasmacytoma (MOPC) 5 to 10 days later, to which intraperitoneally injecting about 10⁶ to 10⁷ cells of hybridoma, and collecting ascites fluid under anesthesia 2 to 5 weeks later, can be mentioned.

### (b) Purification of monoclonal antibody

Separation and purification of the monoclonal antibody are performed according to a method known per se, for example, a method of immunoglobulin separation and purification [e.g., salting-out, alcohol precipitation, isoelectric point precipitation, electrophoresis, adsorption-desorption with an ion exchanger (e.g., DEAE, QEAE), ultracentrifugation, gel filtration, specific purification comprising selectively collecting the antibody by means of an antigen-coupled solid phase or an active adsorbent such as protein A or protein G, and dissociating the linkage to obtain the antibody, and the like].
As described above, a monoclonal antibody can be produced by culturing a hybridoma in or outside the living body of a warm-blooded animal, and harvesting an antibody from the body fluid or culture thereof.

In a preferred mode of embodiment, because the antibody of the present invention is used as a pharmaceutical product having humans as the subject of administration thereof, the antibody in the present invention is an antibody whose risk of showing antigenicity when administered to a human has been reduced; to be specific, the antibody is a fully human antibody, a humanized antibody, a mouse-human chimeric antibody and the like, particularly preferably a fully human antibody. A humanized antibody and a chimeric antibody can be prepared by genetic engineering technology according to the method described below. Although a fully human antibody can also be produced from the above-described human-human (or - mouse) hybridoma, it is desirable to produce it using a human antibody-producing animal described below (e.g., mouse) or the phage display method in order to stably supply the antibody in large amounts at low costs.

### (i) Preparation of chimeric antibody

As used herein, "a chimeric antibody" means an antibody wherein the sequences of the variable regions of the H chain and L chain (V_{H} and V_{L}) thereof are derived from a mammalian species, and wherein the sequences of the constant regions (C_{H} and C_{L}) are derived from another mammalian species. The sequences of the variable regions are preferably derived from, for example, an animal species permitting easy preparation of a hybridoma, such as mouse, and the sequences of the constant regions are preferably derived from the recipient mammalian species.

As examples of the method of preparing a chimeric antibody, the method described in US Patent No. 6,331,415 or a partially modified method thereof and the like can be mentioned. To be specific, first, mRNA or total RNA is prepared from a monoclonal antibody-producing hybridoma (for example, mouse-mouse hybridoma) obtained as described above, according to a conventional method, to synthesize cDNA. DNAs that encode V_{H} and V_{L} are amplified and purified by PCR according to a conventional method with the cDNA as the template, using appropriate primers [for example, oligo DNAs comprising the base sequences that encode the N-terminal sequences of V_{H} and V_{L}, respectively, as the sense primers, and oligo DNAs that hybridize to the base sequences that encode the N-terminal sequences of C_{H} and C_{L}, respectively, as the antisense primer (see, for example, Bio/Technology, 9: 88-89, 1991)]. In the same manner, DNAs that encode C_{H} and C_{L} are amplified and purified from an RNA prepared from lymphocytes and the like of another mammal (e.g., human) by RT-PCR. V_{H} and C_{H}, and V_{L} and C_{L}, are ligated together, respectively, using a conventional method, and the chimeric H chain DNA and chimeric L chain DNA obtained are inserted into respective appropriate expression vectors [for example, vectors comprising promoters that have transcription activity in CHO cells, COS cells, mouse myeloma cells and the like (e.g., CMV promoter, SV40 promoter and the like)]. The DNAs that encode the two chains may be inserted into separate vectors, and may be inserted into a single vector in tandem. Host cells are transformed with the chimeric H chain and chimeric L chain expression vector(s) obtained. As the host cells, animal cells, for example, Chinese hamster ovary (CHO) cells, monkey-derived COS-7 cells, Vero cells, rat-derived GHS cells and the like, in addition to the above-described mouse myeloma cells, can be mentioned. For the transformation, any method applicable to animal cells can be used, with preference given to electroporation method and the like. It is possible to isolate a chimeric monoclonal antibody by culturing the host cells in a medium suitable thereto for a given period, and thereafter recovering the culture supernatant and purifying it in the same manner as described above. Alternatively, it is also possible to obtain a chimeric monoclonal antibody easily and in large amounts from milk or eggs of transgenic animals which are produced by a conventional method using germ line cells of an animal such as bovine, goat, or chicken as the host cells, for which a transgenic technique has been established and a know-how of mass propagation as a domestic animal (domestic fowl) has been compiled. Furthermore, it is also possible to obtain a chimeric monoclonal antibody in large amounts from the seeds, leaves and the like of a transgenic plant, produced by using microinjection and electroporation into protoplast, the particle gun method and Ti-vector method for intact cells and the like, with cells of a plant such as corn, rice, wheat, soybean, or tobacco as the host cells, for which a transgenic technique has been established, and which is cultured in large amounts as a major crop.

When the chimeric monoclonal antibody obtained is digested with papain, Fab is obtained; when the same is digested with pepsin, F(ab')₂ is obtained.
It is also possible to reformat into scFv by ligating DNAs that encode mouse V_{H} and V_{L} via a suitable linker, for example, a DNA that encodes a peptide consisting of 1 to 40 amino acids, preferably 3 to 30 amino acids, more preferably 5 to 20 amino acids [e.g., [Ser-(Gly)m]n or [(Gly)m-Ser]n (m is an integer from 0 to 10, n is an integer from 1 to 5) and the like]. Furthermore, it is possible to reformat into a minibody by ligating a DNA that encodes C_{H3} via a suitable linker thereto, or reformat into a scFv-Fc by ligating a DNA that encodes C_{H} full length via a suitable linker thereto. The DNA encoding such an antibody molecule modified (coupled) by genetic engineering can be expressed in a microorganism such as *E. coli* or yeast under the control of a suitable promoter, to produce the antibody molecule in large amounts.
When DNAs encoding mouse V_{H} and V_{L} are inserted into the downstream of one promoter in tandem and introduced into *E. coli*, a dimer named as Fv is formed by monocistronic gene expression. When an appropriate amino acid in the FRs of V_{H} and V_{L} is substituted with Cys using molecule modeling, a dimer named as dsFv is formed via the intermolecular disulfide bond between the two chains.

### (ii) Humanized antibody

As used herein, "a humanized antibody" means an antibody
wherein the sequences of all regions present in the variable region, other than the complementality determining region (CDR), [i.e., framework region (FR) in constant region and variable region] are derived from a human, and wherein only the sequence of CDR is derived from another mammalian species. The other mammalian species is preferably an animal species, for example, mouse and the like, with which production of hybridomas can be easily performed.

As examples of the method of preparing a humanized antibody, the methods described in US Patent Nos. 5,225,539, 5,585,089, 5,693,761 and 5,693,762 or partially modified methods therefrom and the like can be mentioned. To be specific, DNAs that encode V_{H} and V_{L} derived from a non-human mammalian species (e.g., mouse) are isolated in the same manner as with the above-described chimeric antibody, after which sequencing is performed by a conventional method using an automated DNA sequencer (e.g., manufactured by Applied Biosystems Company and the like), and the base sequences obtained or deduced amino acid sequences therefrom are analyzed using a known antibody sequence database [for example, Kabat database (see Kabat et al., "Sequences of Proteins of Immunological Interest", edited by NIH, US Department of Health and Human Services, Public Health Service, 5th edition, 1991) and the like] to determine the CDR and FR of the two chains. A base sequence wherein the CDR encoding region of a base sequence that encodes the L chain and H chain of a human antibody having an FR sequence similar to the determined FR sequence [e.g., human K type L chain subgroup I and human H chain subgroup II or III (see Kabat et al., 1991 (*supra*))] is substituted with the determined base sequence that encodes the CDR of another animal species, is designed, and the base sequence is divided into fragments of about 20 to 40 bases, and a sequence complementary to the base sequence is divided into fragments of about 20 to 40 bases so that they alternatively overlap with the aforementioned fragments. It is possible to construct DNAs that encode V_{H} and V_{L} having human-derived FR and a CDR derived from another mammalian species by synthesizing individual fragments using a DNA synthesizer, and hybridizing and ligating them in accordance with conventional methods. In order to transfer a CDR derived from another mammalian species into human-derived V_{H} and V_{L} more quickly and more efficiently, it is preferable to use PCR-based site directed mutagenesis. As examples of such a method, the sequential CDR grafting method described in JP-A-5-227970 and the like can be mentioned. It is possible to obtain cells or transgenic animal/plant that produces a humanized antibody by ligating the thus-obtained DNAs that encode V_{H} and V_{L} to DNAs that encode human-derived C_{H} and C_{L}, respectively, in the same manner as with the above-described chimeric antibody, and introducing the ligated product into suitable host cells.

A humanized antibody, like a chimeric antibody, can be modified to scFv, scFv-Fc, minibody, dsFv, Fv and the like by using genetic engineering techniques; and they can be produced in a microorganism such as *E. coli* or yeast by using a suitable promoter.

The technology for preparing a humanized antibody can also be applied to, for example, preparing a monoclonal antibody that can be preferably administered to another animal species for which no hybridoma production technology has been established. For example, animals widely propagated as domestic animals (domestic fowls) such as bovine, swine, sheep, goat, and chicken, and pet animals such as dogs and cats, and the like can be mentioned as the subject animal species.

### (iii) Preparation of fully human antibody using human antibody-producing animal

Provided that a functional human Ig gene is introduced into a non-human warm-blooded animal having the endogenous immunoglobulin (Ig) gene knocked out (KO) therein, and that this animal is immunized with an antigen, a human antibody is produced in place of the antibody derived from the animal. Therefore, provided that an animal such as mice, for which a technique for producing a hybridoma has been established, is used, it is possible to acquire a fully human monoclonal antibody by the same method as the conventional method used to prepare a mouse monoclonal antibody. First, some of the human monoclonal antibodies, that were generated by using a human antibody-producing mouse (see Immunol. Today, 17: 391-397, 1996) obtained by crossing a mouse transfected with minigenes of the human Ig H chain and L chain using an ordinary transgenic (Tg) technique with a mouse wherein the endogenous mouse Ig gene has been inactivated using an ordinary KO technique, are already in clinical stage, and to date production of anti-human Ig human antibody (HAHA) in subject has not been reported.

Later, Abgenix Inc. [trade name: XenoMouse (see Nat. Genet., 15: 146-156, 1997; US Patent No. 5,939,598 and the like)] and Medarex Inc. [trade name: Hu-Mab Mouse (see Nat. Biotechnol., 14: 845-851, 1996; US Patent No. 5,545,806 and the like)] established Tg mice transfected with even a larger human Ig gene using a yeast artificial chromosome (YAC) vector, thus enabling the production of human antibodies of richer repertoire. However, because the human Ig gene, for example, in the case of the H chain, exhibits its diversity as the VDJ exon, which is a variable combination of about 80 kinds of V fragments, about 30 kinds of D fragments and 6 kinds of J fragments, encodes the antigen binding site, the full length thereof is as large as about 1.5 Mb (14th chromosome) for the H chain, about 2 Mb (2nd chromosome) for the KL chain, and about 1 Mb (22nd chromosome) for the λL chain. To reproduce the diverse antibody repertoire in human in another animal species, it is desirable to introduce the full length of each Ig gene. However, a DNA that is insertable into a conventional transfection vector (plasmid, cosmid, BAC, YAC and the like) is normally several kb to several hundred kb in length, and it has been difficult to introduce the full length of Ig genes by the conventional technique for establishing a transgenic animal, which comprises inserting a cloned DNA into a fertilized egg.

Tomizuka et al. (Nat. Genet., 16: 133-143, 1997) prepared a mouse having the full-length human Ig gene by introducing a natural fragment of a human chromosome harboring the Ig gene (hCF) into a mouse [transchromosomic (TC) mouse]. That is, first, a human-mouse hybrid cell having human chromosomes in which the 14th chromosome comprising the H chain gene and the 2nd chromosome comprising the κL chain gene, both labeled with, for example, a drug-resistance marker and the like, is treated with a spindle formation inhibitor (e.g., colcemid) for about 48 hours to prepare a microcell wherein one to several chromosomes or fragments thereof are enveloped in nuclear membrane, and the chromosomes are introduced into a mouse ES cell by the micronuclear fusion method. A hybrid ES cell retaining the chromosomes having the human Ig gene or fragments thereof is selected using a medium containing a drug, and the cell is microinjected into a mouse embryo in the same manner as with the preparation of an ordinary KO mouse. A germ line chimera is selected among the chimeric mice obtained, with coat color as the index, and the like, to establish a TC mouse strain carrying the human 14th chromosome fragment (TC(hCF14)) and a TC mouse strain carrying the human 2nd chromosome fragment (TC(hCF2)). After establishing mouse strains wherein the endogenous H chain gene and KL chain gene are knocked out, respectively [KO (IgH) and KO (Igκ)] by a conventional method, it is possible to establish a mouse strain having all the four kinds of gene modifications (double TC/KO) by repeating the crossing of these four strains.

Provided that the same method as that for producing an ordinary mouse monoclonal antibody is applied to a double TC/KO mouse established as described above, it is possible to obtain an antigen-specific human monoclonal antibody-producing hybridoma. However, there is the drawback of a lower efficiency to obtain hybridomas than that with the ordinary mouse, because hCF2 containing the KL chain gene is unstable in the mouse cells.

On the other hand, because the aforementioned Hu-Mab mouse has a structure wherein the variable region cluster are doubled although it has about 50% of the KL chain gene, it exhibits a K chain diversity equivalent to that with full length (on the other hand, HuMab mouse exhibits a low H chain diversity and inadequate response to antigen because it carries only about 10% of the H chain gene). And the K chain is stably retained in the mouse cells because it is inserted in mouse chromosome via a YAC vector (IgK-YAC). Making use of this advantage, it is possible to get the efficiency for obtaining hybridomas and affinity to antigen affinity of antibody that are equivalent to those with the ordinary mouse, by crossing a TC(hCF14) mouse with a Hu-Mab mouse to establish a mouse that stably retains both hCF14 and Igκ-YAC (trade name: KM mouse).

Furthermore, it is also possible to establish a human antibody-producing animal in which the λL chain gene is further transfected to reconstruct the diverse human antibody repertoire more completely. Such an animal can also be obtained by producing a TC mouse in which the human 22nd chromosome or a fragment thereof harboring the λL chain gene is introduced in the same manner as described above[TC(hCF22)], and crossing the mouse with the above-described double TC/KO mouse or KM mouse, or can also be obtained by, for example, constructing a human artificial chromosome (HAC) comprising both the H chain locus and the λL chain locus, and introducing it into a mouse cell (Nat. Biotechnol., 18: 1086-1090, 2000).

### (iv) Preparation of fully human antibody using phage display human antibody library

Another approach to produce a fully human antibody is a method using phage display. This method sometimes encounters cases in which a mutation due to PCR is introduced into a site other than CDRs; for this reason, a few reports of cases of HAHA production in clinical stage are available. On the other hand, however, the method has advantages such as no risk of cross-species viral infection derived from the host animal and the indefinite specificity of the antibody (antibodies against forbidden clone, sugar chain and the like can also be easily prepared).

The method of preparing a phage display human antibody library include, but are not limited to, for example, the methods described below.
Although a phage used is not subject to limitation, filamentous phage (Ff bacteriophage) is normally preferably used. As the method of presenting a foreign protein on the phage surface, a method comprising expressing and presenting the foreign protein as a fusion protein with any of the coat proteins g3p, and g6p to g9p on the coat protein can be mentioned; and a method comprising fusing the foreign protein to the N-terminal side of g3p or g8p is often used. As the phage display vector, besides 1) one in which the foreign gene is introduced in the form of fusion gene with the coat protein gene of the phage genome, to allow all the coat proteins presented on the phage surface to be presented as a fusion protein with the foreign protein, 2) one in which the gene encoding the fusion protein is inserted separately from the wild-type coat protein gene to allow the fusion protein and the wild-type coat protein to be expressed simultaneously, and 3) an *E. coli* having a phagemid vector harboring the gene that encodes the fusion protein is infected with a helper phage having the wild-type coat protein gene to produce phage particles that express the fusion protein and the wild-type coat protein simultaneously, and the like can be mentioned. However, a phage display vector of the type 2) or 3) is used for the preparation of an antibody library, because in the case of 1), the capability of infection is lost when a large foreign protein is fused.

As a specific vector, those described by Holt et al. (Curr. Opin. Biotechnol., 11: 445-449, 2000) can be mentioned as examples. For example, pCES1 (see J. Biol. Chem., 274: 18218-18230, 1999) is an Fab-expressing phagemid vector wherein a DNA encoding the κL chain constant region allocated to downstream of the g3p signal peptide, and a DNA encoding CH3, His-tag, c-myc tag, and the amber stop codon (TAG) followed by the g3p coding sequence, allocated to downstream of the g3p signal peptide, are arranged under the control of one lactose promoter. When this is introduced to an *E. coli* having an amber mutation, Fab is presented onto the g3p coat protein, but when it is expressed in the HB2151 strain and the like, which do not have an amber mutation, a soluble Fab antibody is produced. And as the scFv-expressing phagemid vector, for example, pHEN1 (J. Mol. Biol., 222: 581-597, 1991) and the like are used.

Meanwhile as examples of the helper phage, M13-KO7, VCSM13 and the like can be mentioned.
And as another phage display vector, a vector that is designed as a DNA sequence comprising the cysteine-encoding codon is linked to each of the 3' end of the antibody gene and the 5' end of the coat protein gene to express the two genes simultaneously and separately (not in the form of a fusion protein), and to present the antibody onto the coat protein on the phage surface via S-S bonds between the introduced cysteine residues (CysDisplay^{™} technology of Morphosys Company) and the like, can be mentioned.

As the kind of human antibody library, a naive/non-immunized library, a synthetic library, an immunized library and the like can be mentioned.
The naive/non-immunized library is a library obtained by acquiring the V_{H} and V_{L} genes retained by a normal human by RT-PCR, and randomly cloning them into the above-described phage display vector. Normally, mRNA derived from lymphocytes of peripheral blood, bone marrow, tonsil and the like of a normal human, and the like are used as the template. A library prepared by selectively amplifying IgM-derived mRNA in which a class switch due to antigen sensitization is not undergoing, to avoid V gene biases such as clinical history, is particularly called a naive library. Representatively, the library of Cambridge Antibody Technology (see J. Mol. Biol., 222: 581-597, 1991; Nat. Biotechnol., 14: 309-314, 1996), the library of Medical Research Council (see Annu. Rev. Immunol., 12: 433-455, 1994), the library of Dyax Corp. (see J. Biol. Chem., 1999 *(supra) ;* Proc. Natl. Acad. Sci. USA, 14: 7969-7974, 2000) and the like can be mentioned.

A synthetic library is obtained by selecting a functional particular antibody gene in human B cells, and substituting a portion of antigen-binding region in a V gene fragment, for example, CDR3 and the like, with DNAs encoding a random amino acid sequence of appropriate length, to construct a library. It is recognized to be excellent in antibody expression efficiency and stability because the library can be constructed with the combination of the V_{H} and V_{L} genes, which produce functional scFv and Fab, since the beginning. Representatively, the HuCAL library of Morphosys AG (see J. Mol. Biol., 296: 57-86, 2000), the library of BioInvent (see Nat. Biotechnol., 18: 852, 2000), the library of Crucell (see Proc. Natl. Acad. Sci. USA, 92: 3938, 1995; J. Immunol. Methods, 272: 219-233, 2003) and the like can be mentioned.

An immunized library is a library obtained by preparing an mRNA from lymphocytes collected from a human such as a patient with cancer, autoimmune disease, infectious disease and the like or a recipient of vaccination, having an elevated blood antibody titer against the target antigen, or from human lymphocytes and the like which are artificially immunized with the target antigen by the above-described *in vitro* immunization method, in the same manner as with the above-described naive/non-immunized library, and amplifying the V_{H} and V_{L} genes by RT-PCR, to construct a library. It is possible to obtain the desired antibody even from such libraries of relatively small size because the desired antibody gene is contained in the library already at the beginning.

The wider the diversity of the library is, the better; actually, however, an appropriate library size is about 10⁸ to 10¹¹ clones, taking into consideration of the number of phages handlable in the following panning operation (10¹¹ to 10¹³ phages) and the number of phages necessary to isolate and amplify clones in ordinary panning (100 to 1,000 phages/clone),; it is possible to screen for an antibody normally having a Kd value on the order of 10⁻⁹ with a library of about 10⁸ clones.

The process for selecting an antibody against the target antigen by the phage display method is referred to as panning. To be specific, for example, a phage presenting an antigen-specific antibody is concentrated by repeating a series of operations of bringing an antigen-immobilized carrier and a phage library into contact with each other, washing out the unbound phage, thereafter eluting the bound phage from the carrier, and infecting the phage to E. *coli* to proliferate it, about 3 to 5 times. As the carrier for immobilizing the antigen, various carriers used in ordinary antigen-antibody reactions or affinity chromatography, for example, insoluble polysaccharides such as agarose, dextran, and cellulose, synthetic resins such as polystyrene, polyacrylamide, and silicon, or microplates, tubes, membranes, columns, beads and the like comprising glass, metal and the like, and surface plasmon resonance (SPR) sensor chips, and the like can be mentioned. For the antigen immobilization, physical adsorption may be used, and a method using a chemical bond used to insolubilize and immobilize a protein or enzyme and the like is also acceptable. For example, a biotin-(strept)avidin system and the like are preferably used. When the endogenous ligand, that is a target antigen, is a small molecule such as a peptide, it is necessary to pay special attention to prevent masking of the portion used as the epitope by conjugating with the carrier. For washing the unbound phage, a blocking solution such as BSA solution (once or twice), a PBS containing a detergent such as Tween (3 to 5 times) and the like can be used. There is also a report mentioning that the use of citrate buffer (pH 5) and the like is preferable for the washing. For elution of the specific phage, an acid (e.g., 0.1 M hydrochloric acid and the like) is normally used; cleavage with a specific protease (for example, a gene sequence that encodes the trypsin cleavage site can be introduced into the linkage site between the antibody gene and the coat protein gene. In this case, *E. coli* infection and proliferation are possible even if all the coat protein is expressed in the form of a fusion protein because the wild-type coat protein is presented on the surface of the eluted phage), competitive elution with a soluble antigen, or elution by reduction of S-S bond (for example, in the aforementioned CysDisplay^{™}, the antigen-specific phage can be recovered by dissociating the antibody and the coat protein by using a suitable reducing agent after performing panning.) is also possible. When elution has been performed with an acid, the eluate is neutralized with Tris and the like, and the eluted phage is then infected to *E. coli,* which is cultured; after which the phage is recovered by a conventional method.

After the phage presenting the antigen-specific antibody is concentrated by panning, the phage is infected to *E. coli* and the cells are sown onto a plate to perform cell cloning. The phage is again collected from the each clone, and the antigen binding activity is confirmed by the antibody titer assay (e.g., ELISA, RIA, FIA and the like) or a measurement utilizing FACS or SPR.

Isolation and purification of the antibody from the selected phage clone that presents the antigen-specific antibody can be performed by, for example, when using a vector incorporating an amber stop codon at the linker site of the antibody gene and the coat protein gene as the phage display vector, infecting the phage to an *E. coli* that does not have amber mutation (e.g., HB2151 strain) to produce and secrete soluble antibody molecules in periplasm or the medium, lysing the cell wall with lysozyme and the like, collecting the extracellular fraction, and purifying using the same purification technique as described above. Provided that the His-tag or c-myc tag has been introduced in advance, the antibody can easily be purified by using IMAC, an anti-c-myc antibody column and the like. When cleavage with a specific protease is utilized in panning, the antibody molecule is separated from the phage surface by an action with the protease, so that the desired antibody can be purified by performing the same purification operation as above mentioned.

The technology for producing a fully human antibody using a human antibody-producing animal and a phage display human antibody library can also be applied to the production of a monoclonal antibody derived from another animal species. For example, animals widely propagated as domestic animals (domestic fowls) such as bovine, swine, sheep, goat, and chicken, and pet animals such as dogs and cats, and the like can be mentioned as the subject animal species. In non-human animals, the utilization of an immunized library is more effective because there are fewer ethical problems concerning artificial immunization with the target antigen.

An antibody of the present invention obtained by any of the methods described above may be one that inhibits the binding of PAC1 to PACAP, or one that does not inhibit the same. Whether or not an antibody of the present invention inhibits the binding of PAC1 to PACAP can be determined by, for example, incubating a solution containing labeled PACAP in contact with a carrier with PAC1 immobilized thereon (may be a PAC1-expressing cell as is or a cell membrane fraction thereof) in the presence and absence of the antibody of the present invention, thereafter removing the liquid phase, measuring the amount of label bound onto the solid phase, and comparing the amount between the two conditions. If the amount of label bound to the solid phase in the presence of the antibody decreases, the antibody can be judged to inhibit the binding of PAC1 to PACAP; if the amount of label does not change, the antibody can be judged not to inhibit the binding of PAC1 to PACAP. Alternatively, also by immobilizing PACAP onto a sensor chip, and measuring and comparing the binding of PAC1 and a PAC1/anti-PAC1 antibody complex to PACAP by surface plasmon resonance, the presence or absence of inhibition of the binding of PAC1 and PACAP can be determined. Also, by allowing labeled PACAP to act on a membrane fraction or a purified receptor in the presence of the antibody, and measuring the amount of label adsorbed along with the receptor to a glass filter, the inhibition of the binding of PAC1 to PACAP by the antibody can be evaluated.
For example, mouse anti-human PAC1 monoclonal antibodies #135E, #130D, 122B and the like described in Examples below inhibit the binding of PAC1 to PACAP, whereas #2C and the like do not inhibit the binding.

Because an antibody of the present invention that inhibits the binding of PAC1 to PACAP blocks the signal transduction of PACAP-PAC1, it is a neutralizing antibody that inhibits the signal transduction activity of PAC1. Whether or not an antibody of the present invention inhibits the signal transduction activity of PAC1 can be determined, more directly, by comparing the PAC1 effector activity regulatory actions in the presence and absence of the antibody. For example, in the presence and absence of the antibody, for example, 1) a method wherein PACAP and ATP are added to a cell containing PAC1 and adenylate cyclase (AC) on the cell membrane thereof or a membrane fraction thereof, and the amount of cAMP produced is measured using an anti-cAMP antibody by a competitive immunoassay with cAMP labeled with a radioactive substance (e.g., ¹²⁵I), an enzyme (e.g., alkaline phosphatase, peroxidase), a fluorescent substance (e.g., FITC, rhodamine) or the like, 2) a method wherein [α-³²P]ATP is added to the above-described cell or membrane fraction thereof, and the [³²P]cAMP produced is separated using an alumina column and the like, thereafter the radioactivity thereof is measured, 3) a method wherein the expression level of a reporter gene (e.g., luciferase gene) under the control of cAMP responding element (CRE) in a transformant cell incorporating the gene is measured, 4) a method wherein [³⁵S]GTPγS (a GTP analogue that does not undergo hydrolysis by the GTPase activity of Gα subunit) is added to a membrane fraction containing PAC1, and the radioactivity bound to the membrane is measured, and the like can be mentioned. Alternatively, in place of 1) to 4) above, 1a) a method
wherein phosphatidylinositol-4,5-diphosphate (PIP) is added to a cell containing PAC1 and phospholipase C (PLC) in the cell membrane thereof or a membrane fraction thereof, and the amount of inositol phosphate (IP3) produced is measured, 2) a method wherein the Ca²⁺ content in a cell containing PAC1 and PLC in the cell membrane thereof is measured, 3) a method
wherein the expression level of a reporter gene under the control of TPA (12-O-tetradecanoylphorbor-13-acetate) responding element (TRE), upregulated by Ca²⁺, in a transformant cell incorporating the gene is measured, and the like can be used. The Ca²⁺ content in the cell can be measured spectroscopically using a fluorescent probe (fura-2, indo-1, fluor-3, Calcium-Green I and the like), or can be measured using aequorin, a calcium-sensitive luminescent protein, and the like. As an apparatus suitable for spectroscopic measurements using a fluorescent probe, the FLIPR (Molecular Devices) system can be mentioned.
For example, mouse anti-human PAC1 monoclonal antibody #130D, described in an Example below, is a complete antibody (IgG) molecule acting as a potent neutralizing antibody that inhibits the GTP-γS binding action of PAC1•G protein more potently with an IC₅₀ on the order of 10⁻⁷ M than the existing peptide antagonist PACAP(6-38)NH₂. A hybridoma that produces this antibody (PC1-SM-130D) has been deposited at the International Patent Organism Depositary (IPOD), National Institute of Advanced Industrial Science and Technology (Chuo 6, 1-1-1, Higashi, Tsukuba, Ibaraki, 305-8566 Japan) since August 4, 2006 (accession number: FERM BP-10655).

Meanwhile, an antibody of the present invention that can be obtained as described above may be one capable of binding to PAC1 antagonistically against PACAP to exhibit the same agonist activity as PACAP, or of binding to an optionally chosen extracellular region of PAC1 to alter the structure of PAC1 to make it in a permanently activated state. Such an antibody can also be screened for as described above.

An antibody of the present invention may be one that inhibits, or does not inhibit, the binding of another anti-PAC1 monoclonal antibody to PAC1. Whether or not an antibody of the present invention inhibits the binding of another anti-PAC1 monoclonal antibody to PAC1 can be determined by, for example, using, instead of PACAP, the other anti-PAC1 monoclonal antibody, in a method mentioned as an example of the above-described testing of the inhibition of the binding of PAC1 and PACAP.
For example, mouse anti-human PAC1 monoclonal antibody #2C, described in an Example below, does not inhibit the binding of PAC1 to PACAP as stated above, but promotes the binding of #34C, #66A and the like to PAC1. Therefore, these antibodies are thought to be capable of neutralizing PAC1 activity more potently when used in combination with #2C than when used alone.

An antibody of the present invention is not only capable of recognizing a PAC1 having a native structure, but also capable of recognizing a denatured PAC1. Therefore, an antibody of the present invention can also be utilized effectively in detecting a denatured PAC1 protein as in

### Western blot analysis.

The present invention provides a PAC1 activity regulator, prevention/treatment of a disease associated with an abnormality of PAC1, and a diagnostic reagent for a disease associated with an abnormality of PAC1 activity, using an antibody of the present invention.

As stated above, an antibody of the present invention is capable of recognizing the native structure of PAC1 and binding thereto to regulate the activity of PAC1, so it can be used as a PAC1 activity regulator. Here, PAC1 activity refers to intracellular signal transduction activity via PAC1 elicited by the binding of PACAP, and to regulate PAC1 activity refers to decreasing or increasing the signal transduction activity.

The biological and pharmacological effects of PACAP and PAC1 in various organs are described in detail in, for example, Pharmacol. Rev., (USA), 2000, Vol.52 (No. 2), pp. 269-324 and the like. For example, PACAP has been suggested as acting as a neurotransmitter and a neuroregulatory factor, as well as acting as a neurotrophic factor and a regulator of neurogenesis, in the central nervous system. Therefore, an antibody of the present invention that enhances a physiological action of PAC1, which is a reporter thereof, can be used as a prophylactic/therapeutic drug for alleviation of cerebral edema and dementic symptoms in the central nervous system and the like. From analyses of mice deficient in the PACAP gene, changes in the responsiveness to various stimuli are observed, and anxiety level reductions and accentuated curiosity are observed; therefore, an antibody of the present invention that inhibits a bioactivity of PAC1, which is a receptor thereof (for example, mouse anti-human PAC1 monoclonal antibodies #135E, #130D, #122B and the like), described in Examples below, can be used as a prophylactic/therapeutic agent for a disease associated with accentuation of a bioactivity of PAC1 in the central nervous system, for example, depression, anxiety and the like.
Meanwhile, analyses of gene-transfected or -deficient mice have shown that PACAP exhibits insulin secretion action and female pregnancy maintenance action in peripheral tissues; therefore, an antibody of the present invention that enhances a bioactivity of PAC1, which is a receptor thereof, can be used as a prophylactic/therapeutic agent for a disease associated with a decrease in a bioactivity of PAC1 in peripheral tissues, for example, diabetes, pregnancy failure and the like.

The antibody of the present invention can be used as the aforementioned prophylactic/therapeutic agent, after being mixed with a pharmacologically acceptable carrier into a pharmaceutical composition, as required.
As the pharmacologically acceptable carrier here, various organic or inorganic carrier substances conventionally used as materials to prepare medicine can be used, which is compounded into as excipient, solvent (dispersing agent), solubilizer, suspending agent, stabilizer, isotonizing agent, buffer, pH adjusting agent, soothing agent and the like. Where necessary, preparation additives such as preservatives, antioxidants and the like can also be used.

As examples of preferable excipients, lactose, sucrose, D-mannitol, D-sorbitol, starch, α-starch, dextrin, crystalline cellulose, low-substituted hydroxypropylcellulose, carboxymethylcellulose sodium, gum arabic, pullulan, light silicic anhydride, synthetic aluminum silicate, magnesium metasilicate aluminate and the like can be mentioned.

As examples of preferable solvents, water for injection, physiological saline, Ringer's solution, alcohols, propylene glycol, polyethylene glycol, sesame oil, corn oil, olive oil, cottonseed oil and the like can be mentioned.

As examples of preferable solubilizers, polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like can be mentioned.

As examples of preferable suspending agents, detergents such as stearyltriethanolamine, sodium lauryl sulfate, laurylaminopropionic acid, lecithin, benzalkonium chloride, benzethonium chloride, and glyceryl monostearate; hydrophilic polymers, for example, polyvinyl alcohol, polyvinylpyrrolidone, carboxymethylcellulose sodium, methylcellulose, hydroxymethylcellulose, hydroxyethylcellulose, hydroxypropylcellulose and the like; polysorbates, polyoxyethylene hydrogenated castor oil and the like can be mentioned.

As examples of preferable stabilizers, human serum albumin (HSA), sodium pyrosulfite, Rongalit, sodium hydrogen metasulfite and the like can be mentioned.

As examples of preferable isotonizing agents, sodium chloride, glycerin, D-mannitol, D-sorbitol, glucose and the like can be mentioned.

As examples of preferable buffers, buffer solutions such as of phosphates, acetates, carbonates and citrates, and the like can be mentioned.

As examples of preferable pH regulators, acids or bases such as hydrochloric acid and sodium chloride can be mentioned.

As examples of preferable soothing agents, benzyl alcohol and the like can be mentioned.

As examples of preferable preservatives, para-oxybenzoic acid esters, chlorobutanol, benzyl alcohol, phenethyl alcohol, dehydroacetic acid, sorbic acid and the like can be mentioned.

As examples of preferable antioxidants, sulfites, ascorbates and the like can be mentioned.

As examples of dosage forms for the above-described pharmaceutical composition, injectable preparations such as injection formulations (e.g., subcutaneous injections, intravenous injections, intramuscular injections, intraperitoneal injections, intra-arterial injections and the like) and drip infusions can be mentioned.

A pharmaceutical composition can be produced by a method in common use in the field of preparation making, for example, a method described in the Japanese Pharmacopoeia and the like. Hereinafter, a specific method of producing preparation is described in detail. The antibody content in the pharmaceutical composition varies depending on dosage form, dose and the like, and is, for example, about 0.1 to 100% by weight.

For example, an injection formulation is produced by dissolving, suspending or emulsifying an antibody, along with a dispersing agent (e.g., polysorbate 80, polyoxyethylene hydrogenated castor oil 60, polyethylene glycol, carboxymethylcellulose, sodium alginate and the like), a preservative (e.g., methylparaben, propylparaben, benzyl alcohol, chlorobutanol, phenol and the like), an isotonizing agent (e.g., sodium chloride, glycerin, D-mannitol, D-sorbitol, glucose and the like) and the like, in an aqueous solvent (e.g., distilled water, physiological saline, Ringer's solution and the like) or an oily solvent (e.g., vegetable oils such as olive oil, sesame oil, cottonseed oil and corn oil, propylene glycol and the like). If desired, additives such as a solubilizer (e.g., sodium salicylate, sodium acetate and the like), a stabilizer (e.g., human serum albumin and the like), and a soothing agent (e.g., benzyl alcohol and the like) may be used. The injectable solution is subjected to a sterilizing treatment, for example, filtration sterilization using a membrane filter and the like as required, and is usually filled in appropriate containers such as ampoules.

The injection formulation can also be used as a fresh supply obtained by dissolving (dispersing) a powder prepared by treating the above-described liquid by vacuum drying and the like. Examples of methods of vacuum drying include lyophilization and a method using the Speedback Concentrator (SAVANT Company). When performing lyophilization, it is preferable to lyophilize the sample, cooled below -10°C, using a flask in the laboratory or a tray or vial in industrial settings. When the Speedback Concentrator is used, lyophilization is performed at about 0 to 30°C under a vacuum of about 20 mmHg or less, preferably about 10 mmHg or less. It is preferable to add a buffer such as a phosphate to the liquid to be dried, to obtain a pH of about 3 to 10. The powder preparation obtained by lyophilization, as a long-stable preparation, can be prepared freshly as an injection formulation by dissolving in water for injection, physiological saline, Ringer's solution and the like, or by dispersing in olive oil, sesame oil, cottonseed oil, corn oil, propyleneglycol and the like before use.

Furthermore, an antibody of the present invention may be used in combination with other drugs and the like. For example, when an antibody that inhibits a bioactivity of PAC1 is used as a prophylactic/therapeutic agent for depression, anxiety and the like, other drugs possessing prophylactic/therapeutic activity for the disease, for example, anti-depression drugs such as imipramine, clomipramine, amitriptyline, maprotiline, fluvoxamine, paroxetine, fluoxetine, and milnacipran, and anti-anxiety drugs such as chlorodiazepoxide, diazepam, oxazepam, bromazepam, lorazepam, alprazolam, clotiazepam, and etizolam, and the like can be used in combination. Also, for example, when an antibody that enhances a bioactivity of PAC1 is used as a prophylactic/therapeutic agent for cerebral edema, dementic symptoms, diabetes, pregnancy failure and the like, other drugs possessing prophylactic/therapeutic activity for the disease, for example, anti-cerebral edema drugs such as glycerol, fructose, mannitol, and steroids; dementic symptom ameliorators such as donepezil, amantadine, and tiapride; anti-diabetic drugs such as insulin, tolbutamide, glyclopyramide, glibenclamide, metformin, epalrestat, voglibose, acarbose, and troglitazone; and therapeutic drugs for pregnancy failure such as clomifene citrate, Sexovid, human menopausal gonadotropin (HMG), and human chorionic gonadotropin (HCG), and the like can be used in combination.

When an antibody of the present invention and another drug described above are used in combination, for example (1) they may be produced as a single formulation, along with an excipient and the like that are acceptable for pharmaceutical making, added as desired for pharmaceutical making, in accordance with a publicly known method of pharmaceutical making, (2) they may be produced as respective preparations, along with an excipient and the like that are acceptable for pharmaceutical making, added as desired, and used in combination (coadministered) at the same time or at a time lag, or (3) they may be produced as respective preparations, along with an excipient as appropriate, by a conventional method, and used in a set (kit formulation and the like) and the like. In case (2), as far as an object of the present invention is accomplished, the dosing frequency may be different among the individual preparations. The active ingredient content in such a preparation may be any one falling in the range of effective amounts of each active ingredient or in a pharmaceutically and pharmacologically acceptable range. Specifically, the content is normally about 0.01 to about 100% by weight.
Alternatively, by joining an antibody of the present invention and another drug described above, via an appropriate linker as required, to obtain an immunoconjugate, the antibody of the present invention not only acts as a prophylactic/therapeutic drug, but also has an effect to target the concomitant drug to a target cell (i.e., PAC1-expressing cell).

The dose of the above-described preparation containing an antibody of the present invention varies depending on the subject of administration, target disease, symptoms, route of administration and the like; for example, when the preparation is used for the prevention/treatment of depression, it is convenient to administer it usually in an amount of about 0.01 to about 20 mg/kg body weight, preferably about 0.1 to about 10 mg/kg body weight, more preferably about 0.1 to about 5 mg/kg body weight, based on the amount of an antibody of the present invention per dose, about once every 3 weeks to about twice a week, preferably once every 2 weeks to about once a week, by intravenous injection, preferably intravenous drip infusion. In the case of intravenous drip infusion, each dose is given, for example, over about 30 to about 180 minutes, preferably about 60 to about 120 minutes. In the case of other parenteral administration and oral administration, similar doses may be administered. In case of a particularly severe symptom, the dose may be increased according to the symptom.
When an antibody of the present invention and another drug are used in combination, a dose can be chosen as appropriate according to the subject of administration, the age and body weight of the subject of administration, symptoms, duration of administration, method of administration, dosage form, drug combination and the like on the basis of the minimum recommended clinical dose of each drug.

Because an antibody of the present invention is capable of specifically recognizing PAC1, it can be used for detection and quantitation of PAC1 in a test liquid, particularly for detection and quantitation by sandwich immunoassay and the like. Hence, the present invention provides, for example, (i) a method of quantitating PAC1 in a test liquid, comprising competitively reacting an antibody of the present invention and a test liquid and a labeled protein and the like, and measuring the ratio of labeled protein and the like bound to the antibody, and (ii) a method of quantitating PAC1 in a test liquid, comprising simultaneously or sequentially reacting the test liquid and an antibody of the present invention insolubilized on a carrier, and a labeled antibody of the present invention, thereafter measuring the activity of the labeling agent on the insolubilizing carrier.
In (ii) above, it is preferable that the insolubilized antibody and the labeled antibody have an antigen recognition site that does not interfere with the binding with PAC1 (for example, one antibody recognizes an N-terminal portion of PAC1, and the other antibody recognizes an extracellular loop portion, and the like).

Because an antibody of the present invention is capable of recognizing a PAC1 having a native structure, it enables in situ detection by tissue staining and the like. For these purposes, the antibody molecule itself may be used, and any fragment thereof, such as the F(ab')2, Fab' or Fab fraction of the antibody molecule, may also be used. The method for measurement using an antibody against PAC1 is not to be limited particularly; any method of measurement can be used, as far as the amount of antibody, antigen or antibody-antigen complex corresponding to the amount of antigen in a test liquid is detected by a chemical or physical means, and calculated from a standard curve generated using standard solutions containing known amounts of the antigen. For example, nephelometry, the competitive method, the immunometric method and the sandwich method are suitably used, and the sandwich method described below is particularly preferable in terms of sensitivity and specificity.

As the labeling agent to be used for the measurement method using a labeling substance, for example, a radioisotope, an enzyme, a fluorescent substance, a luminescent substance and the like can be used. As the radioisotope, for example, [¹²⁵I], [¹³¹I], [³H], [¹⁴C] and the like can be used. The above-described enzyme is preferably stable and has a high specific activity and, for example, β-galactosidase, β-glucosidase, alkaline phosphatase, peroxidase, malate dehydrogenase and the like can be used. As the fluorescent substance, for example, fluorescamine, fluorescein isothiocyanate and the like can be used. As the luminescent substance, for example, luminol, luminol derivative, luciferin, lucigenin and the like can be used.

For insolubilization of the antigen or antibody, physical adsorption may be used, and methods of chemical binding for conventional use to insolubilize or immobilize proteins, enzymes and the like may be used as well. As examples of the carrier, insoluble polysaccharides such as agarose, dextran, and cellulose; synthetic resins such as polystyrene, polyacrylamide, and silicon, or glass and the like can be mentioned.
In the sandwich method, an antibody of the present invention insolubilized is reacted with a test liquid (primary reaction), then reacted with the antibody of the present invention labeled (secondary reaction), after which the activity of the labeling agent on the insolubilizing carrier is measured, whereby the amount of PAC1 in the test liquid can be quantified. The primary reaction and the secondary reaction may be performed in the reverse order, or performed simultaneously, or performed with a time lag. The labeling agent and the method for insolubilization can be the same as those described above.

In the immunoassay by the sandwich method, the antibody used for the solid phase antibody or the antibody for labeling is not necessarily from one kind, but a mixture of two or more kinds of antibodies may be used to increase the measurement sensitivity and for other purposes.
In the method of measuring PAC1 by the sandwich method, antibodies of the present invention used in the primary reaction and the secondary reaction are preferably antibodies whose portions involved in binding to PAC1 are mutually different. Hence, for the antibodies used in the primary reaction and the secondary reactions, for example, when the antibody used in the secondary reaction recognizes the N-terminus of PAC1, the antibody used in the primary reaction is an antibody that recognizes a portion other than the N-terminus, for example, an extracellular loop portion.

An antibody of the present invention can be used for measurement systems other than the sandwich method, for example, the competitive method, immunometric method, nephelometry and the like. In the competitive method, the antigen in a test liquid and labeled antigen are competitively reacted with an antibody, after which the unreacted labeled antigen (F) and the labeled antigen bound to the antibody (B) are separated (B/F separation), the amount of the labeled antigen in B or F is measured, and the amount of antigen in the test liquid is quantified. This reaction method includes a liquid phase method using a soluble antibody as an antibody, polyethylene glycol and a secondary antibody to the soluble antibody for B/F disjoining, and an immobilization method using an immobilized antibody as the primary antibody, or using a soluble antibody as the primary antibody and an immobilized antibody as the secondary antibody.
In the immunometric method, the antigen in a test liquid and a solid-phase-immobilized antigen are competitively reacted with a given amount of a labeled antibody, after which the solid phase and the liquid phase are separated, or the antigen in the test liquid and an excess amount of the labeled antibody are reacted, and then a solid-phase-immobilized antigen is added to bind the unreacted portion of the labeled antibody to the solid phase, after which the solid phase and the liquid phase are separated. Next, the amount of labeling agent in either phase is measured to quantify the amount of antigen in the test liquid.
Also, in nephelometry, the amount of insoluble precipitate resulting from an antigen-antibody reaction in the gel or in the solution is measured. Even when the amount of antigen in the test liquid is small and only a small amount of precipitate is obtained, laser nephelometry, which utilizes laser scattering, and the like are suitably used.

In applying these individual immunological measurement methods to the quantitation of PAC1, it is unnecessary to set special conditions, procedures and the like. Making ordinary technical considerations for those skilled in the art to the ordinary conditions and procedures in each method, a measurement system for PAC1 can be constructed. For details of these general technical means, compendia, books and the like can be referred to [see, for example, edited by Hiroshi Irie, "Rajioimunoassei" (Kodansha, published in 1974), edited by Hiroshi Irie, "Zoku Rajioimunoassei" (Kodansha, published in 1979), edited by Eiji Ishikawa et al., "Kouso Meneki Sokuteihou" (Igaku-Shoin, published in 1978), edited by Eiji Ishikawa et al., "Kouso Meneki Sokuteihou" (2nd edition) (Igaku-Shoin, published in 1982), edited by Eiji Ishikawa, "Kouso Meneki Sokuteihou" (3rd edition) (Igaku-Shoin, published in 1987), "Methods in ENZYMOLOGY", Vol. 70 (Immunochemical Techniques (Part A)), ibidem, Vol. 73 (Immunochemical Techniques (Part B)), ibidem, Vol. 74 (Immunochemical Techniques (Part C)), ibidem, Vol. 84 (Immunochemical Techniques (Part D: Selected Immunoassays)), ibidem, Vol. 92 (Immunochemical Techniques (Part E: Monoclonal Antibodies and General Immunoassay Methods)), ibidem, Vol. 121 (Immunochemical Techniques (Part I: Hybridoma Technology and Monoclonal Antibodies)) (all published by Academic Press) and the like].
Using an antibody of the present invention as described above, PAC1 can be quantified highly sensitively.
Furthermore, by quantifying PAC1 in a living organism using an antibody of the present invention, various diseases associated with accentuation or decrease in PAC1 activity can be diagnosed.
An antibody of the present invention can be used to specifically detect PAC1 present in a sample such as a body fluid or tissue. An antibody of the present invention can also be used for preparation of an antibody column used to purify PAC1, detection of PAC1 in each fraction during purification, analysis of the behavior of PAC1 in a subject cell and the like.

Using an antibody of the present invention as described above makes it possible to diagnose a disease associated with an abnormality (accentuation or reduction) of PAC1 activity, for example, depression, anxiety, mental behavioral dysfunction, sexual dysfunction, (circadian rhythm photosynchronization disorder), cerebral edema, dementic symptoms, insulin secretion disorders such as diabetes and hyperinsulinemia, pregnancy failure and the like in a subject animal (for example, human, horse, dog, cat, monkey, cattle, pig, sheep, goat, rabbit, mouse, rat, hamster, guinea pig, chicken and the like). More specifically, the present invention provides a diagnostic method for a disease associated with an abnormality of PAC1 activity by measuring the amount of PAC1 expressed in a biological sample using an antibody of the present invention. Here, "diagnosis" is used not only to mean determining the presence or absence of onset, but also as a meaning encompassing predicting whether or not the disease is likely to onset in the future, evaluating the degree of progression of a pathologic condition that has already developed in a subject animal and the like.

Any biological sample can be used in the present invention, as far as it is derived from a subject animal; for example, humoral fluids (e.g., blood, plasma, serum, lymph, cerebrospinal fluid, synovial fluid, semen, urine, sweat, tears and the like), various cells/tissue sections and the like can be mentioned, and a humoral fluid is preferable because of its low invasion to the subject animal, but, taking into account the fact that PAC1 is a membrane protein, the sample is desirably a cell-containing sample. Therefore, a cell or tissue section (biopsy sample) from a site suspected of being affected by a disease, or blood, blood cells and the like are preferable.

Quantitation of PAC1 in a biological sample can be performed by any method described above. If a result of a measurement shows that the PAC1 content has increased significantly compared with normal values, the subject animal can be diagnosed as being likely to have a disease associated with accentuation of PAC1 activity, or being likely to suffer from the disease in the future. Conversely, if the PAC1 content has decreased significantly compared with normal values, the subject animal can be diagnosed as being likely to have a disease associated with a decrease in PAC1 activity, or being likely to suffer from the disease in the future.

As stated above, an antibody of the present invention can be used to quantify PAC1, therefore it is useful as a reagent for screening for a substance that regulates the expression of PAC1.
Hence, the present invention provides a screening method for a substance that regulates the expression of PAC1, which comprises using an antibody of the present invention and a PAC1-expressing cell, and a substance obtained by the screening method.

Hence, the present invention provides, for example, (i) a screening method for a substance that regulates the expression of PAC1, comprising disrupting a tissue, cells or the like isolated from (1) blood, (2) a particular organ, or (3) an organ of a non-human mammal, then isolating a cell membrane fraction, and quantifying the PAC1 contained in the cell membrane fraction, (ii) a screening method for a substance that regulates the expression of PAC1, comprising disrupting a transformant that expresses PAC1 or a partial peptide thereof and the like, then isolating a cell membrane fraction, and quantifying the PAC1 contained in the cell membrane fraction, (iii) a screening method for a substance that regulates the expression of PAC1, comprising sectioning (1) blood, (2) a particular organ, (3) or a tissue or cell isolated from an organ, and the like from a non-human mammal, and using an immunological staining method to quantify the degree of staining of PAC1 on the cell surface layer to confirm, (iv) a screening method for a substance that regulates the expression of PAC1, comprising sectioning a transformant that expresses PAC1 or a partial peptide thereof and the like, and using an immunological staining method to quantify the degree of staining of PAC1 on the cell surface layer to confirm PAC1 on the cell membrane.

Quantitation of PAC1 contained in a cell membrane fraction is specifically performed as described below.
(i) A drug (for example, anti-obesity drugs, anti-diabetic drugs, antihypertensive drugs, vasoactive drugs, antidepressive drugs and the like) or a physical stress (for example, soaking stress, electric shock, brightness/darkness, low temperatures and the like) or the like is given to a normal or disease model non-human mammal (for example, mouse, rat, rabbit, sheep, pig, cattle, cat, dog, monkey and the like, more specifically, obese mouse, diabetic mouse, hypertensive rat, arteriosclerotic rabbit, depressive rat and the like); after a given time has elapsed, blood or a particular organ (for example, pancreas, pituitary and the like), a tissue (for example, pancreatic islet and the like) or cells (for example, pancreatic β cells, pituitary glandular cells and the like) are obtained. The cells and the like obtained are suspended in, for example, an appropriate buffer solution (for example, Tris-HCl buffer solution, phosphate buffer solution, HEPES buffer solution and the like) and the like, and the cells or the like are disrupted using a detergent (for example, Triton X100^{™}, Tween 20^{™} and the like) and the like, and further treated using techniques such as centrifugation, filtration and column fractionation to yield a cell membrane fraction.

A cell membrane fraction refers to a fraction rich in the cell membrane, obtained by a method known per se after cell disruption. As the method of cell disruption, cell squashing using a Potter-Elvehjem homogenizer, disruption using a Waring blender or Polytron (manufactured by Kinematica Inc.), disruption by sonication, disruption by cell spraying through a thin nozzle under an increased pressure using a French press or the like, and the like can be mentioned. For cell membrane fractionation, fractionation by centrifugal forces, such as fractional centrifugation or density gradient centrifugation, is mainly used. For example, a cell disruption fluid is centrifuged at a low speed (500 rpm to 3000 rpm) for a short time (normally about 1 to 10 minutes), the supernatant is centrifuged at a higher speed (15000 rpm to 30000 rpm) normally for 30 minutes to 2 hours, and the precipitate obtained is used as the membrane fraction. The membrane fraction is rich in PAC1 and cell-derived membrane components such as phospholipids and membrane protein.

The PAC1 contained in the cell membrane fraction can be quantified by, for example, a sandwich immunoassay, Western blot analysis and the like using an antibody of the present invention.
Such a sandwich immunoassay can be performed in the same manner as the above-described method, and Western blot can be performed by a means known per se.

(ii) A transformant that expresses PAC1 or a partial peptide thereof may be prepared in accordance with the above-described method, and the PAC1 contained in the cell membrane fraction can be quantified.

Screening for a substance that regulates the expression of PAC1 can be performed by:
(i) administering a test compound to a normal or disease model non-human mammal at a given time before (before 30 minutes to before 24 hours, preferably before 30 minutes to before 12 hours, more preferably before 1 hour to before 6 hours) or at a given time after (after 30 minutes to after 3 days, preferably after 1 hour to after 2 days, more preferably after 1 hour to after 24 hours) a drug, a physical stress or the like is given, or at the same time as the drug or physical stress is given, and quantifying the amount of PAC1 in the cell membrane after a given time has elapsed after administration (after 30 minutes to after 3 days, preferably after 1 hour to after 2 days, more preferably after 1 hour to after 24 hours), and can also be preformed by:
(ii) mixing a test compound in a medium at the start of cultivation of a transformant according to a conventional method, and quantifying the amount of PAC1 in the cell membrane after cultivation for a given time (after 1 day to after 7 days, preferably after 1 day to after 3 days, more preferably after 2 days to after 3 days).

Identification of the PAC1 contained in the cell membrane fraction is specifically performed as described below.
(iii) A drug (for example, anti-obesity drug, anti-diabetic drug, antihypertensive drug, vasoactive drug, anti-depressive drug and the like) or a physical stress (for example, soaking stress, electric shock, brightness/darkness, low temperatures and the like) or the like is given to a normal or disease model non-human mammal (for example, mouse, rat, rabbit, sheep, pig, cattle, cat, dog, monkey and the like, more specifically, obese mouse, diabetic mouse, hypertensive rat, arteriosclerotic rabbit, depressive rat and the like); after a given time has elapsed, blood or a particular organ (for example, pancreas, pituitary and the like), a tissue (for example, pancreatic islet and the like) or cells (for example, pancreatic β cells, pituitary glandular cells and the like) are obtained. The cells or the like obtained are cut into tissue sections according to a conventional method, and immunostaining is performed using an antibody of the present invention. By quantifying the extent of staining of PAC1 on the cell surface layer to identify the PAC1 on the cell membrane, the amount of PAC1 in the cell membrane can be confirmed quantitatively or qualitatively.
(iv) Identification is also possible in a similar way using a transformant that expresses PAC1 or a partial peptide thereof and the like.

A substance obtained using the above-described screening method is a compound possessing an activity to regulating the expression of PAC1 (altering the amount of PAC1 in the cell membrane), specifically (a) a substance that enhances a cell stimulation activity mediated by a PACAP-PAC1 interaction (for example, GTP-GDP exchange reaction promotion in conjugated Gα, intracellular cAMP production promotion, inositol phosphate production promotion, intracellular Ca²⁺ uptake promotion, insulin secretion promotion and the like) by increasing the expression of PAC1 (increasing the amount of PAC1 in the cell membrane), (b) or a substance that weakens the cell stimulation activity by decreasing the expression of PAC1 (decreasing the amount of PAC1 in the cell membrane).
As the substance, peptides, proteins, non-peptide compounds, synthetic compounds, fermentation products and the like can be mentioned; these substances may be novel substances or publicly known substances.
A substance that enhances the cell stimulation activity is useful as a safe and less toxic pharmaceutical for enhancing a bioactivity of PAC1.
A substance that weakens the cell stimulation activity is useful as a safe and less toxic pharmaceutical for reducing a bioactivity of PAC1.

A substance that regulates the expression of PAC1 can be used as the above-described pharmaceutical after being mixed with a pharmacologically acceptable carrier to obtain a pharmaceutical composition as required.
Here, as the pharmacologically acceptable carrier, various organic or inorganic carrier substances in common use as pharmaceutical materials can be used, which are formulated as excipients, lubricants, binders, and disintegrants in solid preparations; as solvents, solubilizers, suspending agents, isotonizing agents, buffers, soothing agents and the like in liquid preparations. Pharmaceutical additives such as antiseptics, antioxidants, colorants, and sweeteners can also be used as necessary.
As examples of preferable excipient, lactose, sucrose, D-mannitol, D-sorbitol, starch, α-starch, dextrin, crystalline cellulose, low-substituted hydroxypropylcellulose, sodium carboxymethylcellulose, gum arabic, pullulan, light anhydrous silicic acid, synthetic aluminum silicate, magnesium alumino metasilicate and the like can be mentioned.
As examples of preferable lubricants, magnesium stearate, calcium stearate, talc, colloidal silica and the like can be mentioned.
As examples of preferable binders, α-starch, cane sugar, gelatin, gum arabic, methylcellulose, carboxymethylcellulose, carboxymethylcellulose sodium, crystalline cellulose, sucrose, D-mannitol, trehalose, dextrin, pullulan, hydroxypropylcellulose, hydroxypropylmethylcellulose, polyvinylpyrrolidone and the like can be mentioned.
As examples of suitable disintegrants, lactose, sucrose, starch, carboxymethylcellulose, carboxymethylcellulose calcium, crosslinked carmellose sodium, carboxymethylstarch sodium, light silicic anhydride, low-substituted hydroxypropylcellulose and the like can be mentioned.
As examples of preferable solvents, water for injection, physiological saline, Ringer's solution, alcohol, propylene glycol, polyethylene glycol, sesame oil, corn oil, olive oil, cottonseed oil and the like can be mentioned.
As examples of preferable solubilizers, polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like can be mentioned.
As examples of preferable suspending agents, detergents such as stearyltriethanolamine, sodium lauryl sulfate, laurylaminopropionic acid, lecithin, benzalkonium chloride, benzethonium chloride, and glyceryl monostearate; hydrophilic polymers such as polyvinyl alcohol, polyvinylpyrrolidone, carboxymethylcellulose sodium, methylcellulose, hydroxymethylcellulose, hydroxyethylcellulose, and hydroxypropylcellulose; polysorbates, polyoxyethylene hydrogenated castor oil and the like can be mentioned.
As examples of preferable isotonizing agents, sodium chloride, glycerin, D-mannitol, D-sorbitol, glucose and the like can be mentioned.
As examples of preferable buffers, buffer solutions such as of phosphates, acetates, carbonates and citrates, and the like can be mentioned.
As examples of preferable soothing agents, benzyl alcohol and the like can be mentioned.
As examples of preferable preservatives, p-hydroxybenzoic esters, chlorobutanol, benzyl alcohol, phenethyl alcohol, dehydroacetic acid, sorbic acid and the like can be mentioned.
As examples of preferable antioxidants, sulfites, ascorbic acid salts and the like can be mentioned.
As examples of suitable colorants, water-soluble food tar colors (e.g., food colors such as Food Color Red No.2 and No.3, Food Color Yellow No. 4 and No.5, and Food Color Blue No. 1 and No.2), water-insoluble lake colors (e.g., aluminum salts of the aforementioned water-soluble food tar colors, and the like), natural colors (e.g., β-carotene, chlorophyll, red iron oxide and the like) and the like can be mentioned.
As examples of preferable sweeteners, saccharin sodium, dipotassium glycyrrhizinate, aspartame, stevia and the like can be mentioned.

As examples of dosage forms for the aforementioned pharmaceutical composition, oral formulations such as tablets, capsules (including soft capsules and microcapsules), granules, powders, syrups, emulsions, and suspensions; and non-oral formulations such as injection formulations (e.g., subcutaneous injections, intravenous injections, intramuscular injections, intraperitoneal injections, etc.), external formulations (e.g., nasal preparations, transdermal preparations, ointments and the like), suppositories (e.g., rectal suppositories, vaginal suppositories and the like), pellets, drip infusions, and sustained-release preparations (e.g., sustained-release microcapsules and the like) can be mentioned.
These pharmaceutical compositions can be produced by a method in common use in the field of drug formulation technology, for example, methods described in the Japanese Pharmacopoeia and the like. Specific methods of preparing preparations are described in detail below. The active ingredient content in the pharmaceutical composition varies depending on dosage form, dose of the active ingredient and the like, and is, for example, about 0.1 to 100% by weight.
For example, an oral formulation is produced by adding an excipient (e.g., lactose, sucrose, starch, D-mannitol and the like), a disintegrant (e.g., carboxymethylcellulose calcium and the like), a binder (e.g., α-starch, gum arabic, carboxymethylcellulose, hydroxypropylcellulose, polyvinylpyrrolidone and the like), or a lubricant (e.g., talc, magnesium stearate, polyethylene glycol 6000 and the like) and the like, to an active ingredient, and compressing them, with coating performed, if necessary, using a coating base for the purpose of taste masking, enteric dissolution or sustained release, by a method known per se.

As examples of the coating base, sugar-coating bases, water-soluble film-coating bases, enteric film-coating bases, sustained-release film-coating bases and the like can be mentioned.
As the sugar-coating base, sucrose is used, which may be used in combination with one kind or two or more kinds selected from among talc, precipitated calcium carbonate, gelatin, gum arabic, pullulan, carnauba wax and the like.
As examples of the water-soluble film-coating base, cellulose polymers such as hydroxypropylcellulose, hydroxypropylmethylcellulose, hydroxyethylcellulose and methylhydroxyethylcellulose; synthetic polymers such as polyvinylacetal diethylaminoacetate, aminoalkylmethacrylate copolymer E [Eudragit E (trade name), Roehm Pharma GmbH], and polyvinylpyrrolidone; polysaccharides such as pullulan; and the like can be mentioned.
As examples of the enteric film-coating base, cellulose polymers such as hydroxypropylmethylcellulose phthalate, hydroxypropylmethylcellulose acetate succinate, carboxymethylethylcellulose, and cellulose acetate phthalate; acrylate polymers such as methacrylate copolymer L [Eudragit L (trade name), Roehm Pharma GmbH], methacrylate copolymer LD [Eudragit L-30D55 (trade name), Roehm Pharma GmbH], and methacrylate copolymer S [Eudragit S (trade name), Roehm Pharma GmbH]; naturally occurring substances such as shellac; and the like can be mentioned.
As examples of the sustained-release film-coating base, cellulose polymers such as ethylcellulose; acrylate polymers such as aminoalkylmethacrylate copolymer RS [Eudragit RS (trade name), Roehm Pharma GmbH] and ethyl acrylate/methyl methacrylate copolymer suspension [Eudragit NE (trade name), Roehm Pharma GmbH]; and the like can be mentioned.
The above-described coating bases may be used in a suitable mixture of two or more kinds thereof. Also, a light-blocking agent such as titanium oxide or iron sesquioxide may be used during coating.

For example, an injection is produced by dissolving, suspending or emulsifying active ingredient, along with a dispersing agent (e.g., polysorbate 80, polyoxyethylene hydrogenated castor oil 60, polyethylene glycol, carboxymethylcellulose, sodium alginate and the like), a preservative (e.g., methylparaben, propylparaben, benzyl alcohol, chlorobutanol, phenol and the like), an isotonizing agent (e.g., sodium chloride, glycerin, D-mannitol, D-sorbitol, glucose and the like) and the like, in an aqueous solvent (e.g., distilled water, physiological saline, Ringer's solution and the like) or an oily solvent (e.g., vegetable oils such as olive oil, sesame oil, cottonseed oil and corn oil, propylene glycol and the like). If desired, additives such as a solubilizer (e.g., sodium salicylate, sodium acetate and the like), a stabilizer (e.g., human serum albumin and the like), and a soothing agent (e.g., benzyl alcohol and the like) may be used. The injection is usually filled in a suitable ampoule.

Because the preparation thus obtained is safe and less toxic, it can be administered to, for example, mammals (for example, humans, rats, rabbits, sheep, pigs, cattle, cats, dogs, monkeys and the like).

The dose of a substance that regulates the expression of PAC1 varies depending on the subject of administration, target organ, symptoms, method of administration and the like; in the case of oral administration, the dose is normally about 0.1 to 100 mg, preferably about 1.0 to 50 mg, and more preferably about 1.0 to 20 mg, per day, in, for example, a depressive patient (assuming a 60 kg body weight). In the case of parenteral administration, the single dose varies depending on the subject of administration, target organ, symptoms, method of administration and the like; for example, in the form of an injection formulation, it is advantageous that the dose is normally about 0.01 to 30 mg, preferably about 0.1 to 20 mg, and more preferably about 0.1 to 10 mg, per day, in, for example, a depressive patient (assuming a 60 kg body weight). In cases where the subject of administration is other than a human, a dose corresponding to a human dose per 60 kg body weight can be administered.

In the present specification and drawings, the codes of bases, amino acids and the like are denoted in accordance with the IUPAC-IUB Commission on Biochemical Nomenclature or by the common codes in the art. Examples thereof are as follows. For amino acids that may have optical isomers, the L form is presented unless otherwise indicated.
- DNA :: deoxyribonucleic acid
- cDNA :: complementary deoxyribonucleic acid
- A :: adenine
- T :: thymine
- G :: guanine
- C :: cytosine
- RNA :: ribonucleic acid
- mRNA :: messenger ribonucleic acid
- dATP :: deoxyadenosine triphosphate
- dTTP :: deoxythymidine triphosphate
- GTP :: deoxyguanosine triphosphate
- dCTP :: deoxycytidine triphosphate
- ATP: : adenosine triphosphate
- EDTA :: ethylenediamine tetraacetic acid
- SDS: : sodium dodecyl sulfate
- Gly: : glycine
- Ala: : alanine
- Val: : valine
- Leu: : leucine
- Ile: : isoleucine
- Ser: : seine
- Thr: : threonine
- Cys: : cysteine
- Met: : methionine
- Glu: : glutamic acid
- Asp: : aspartic acid
- Lys: : lysine
- Arg: : arginine
- His: : histidine
- Phe: : phenylalanine
- Tyr: : tyrosine
- Trp: : tryptophan
- Pro: : proline
- Asn: : asparagine
- Gln: : glutamine
- pGlu :: pyroglutamic acid
- Me: : methyl group
- Et: : ethyl group
- Bu: : butyl group
- Ph: : phenyl group
- TC: : thiazolidin-4(R)-carboxamide group

Furthermore, substituents, protecting groups and reagents often used in the present description are denoted using the following symbols:
- Tos :: p-toluenesulfonyl
- CHO :: formyl
- Bzl :: benzyl
- Cl₂Bzl :: 2,6-dichlorobenzyl
- Bom :: benzyloxymethyl
- Z :: benzyloxycarbonyl
- Cl-Z :: 2-chlorobenzyloxycarbonyl
- Br-Z :: 2-bromobenzyloxycarbonyl
- Boc :: t-butoxycarbonyl
- DNP :: dinitrophenol
- Trt :: trityl
- Bum :: t-butoxymethyl
- Fmoc :: N-9-fluorenyl methoxycarbonyl
- HOBt :: 1-hydroxybenztriazole
- HOOBt :: 3,4-dihydro-3-hydroxy-4-oxo-1,2,3-benztriazine
- HONB :: 1-hydroxy-5-norbornene-2,3-dicarboximide
- DCC :: N,N'-dicyclohexylcarbodiimide

The present invention is hereinafter described in further detail by means of the following examples, which, however, do not limit the scope of the present invention.

### [Examples]

### Example 1 Purification of human PAC1 using SM1200/CHS mixed micelle system

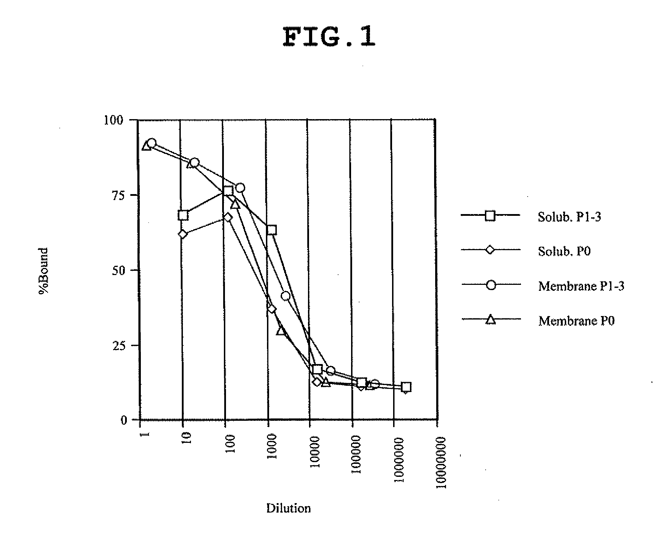

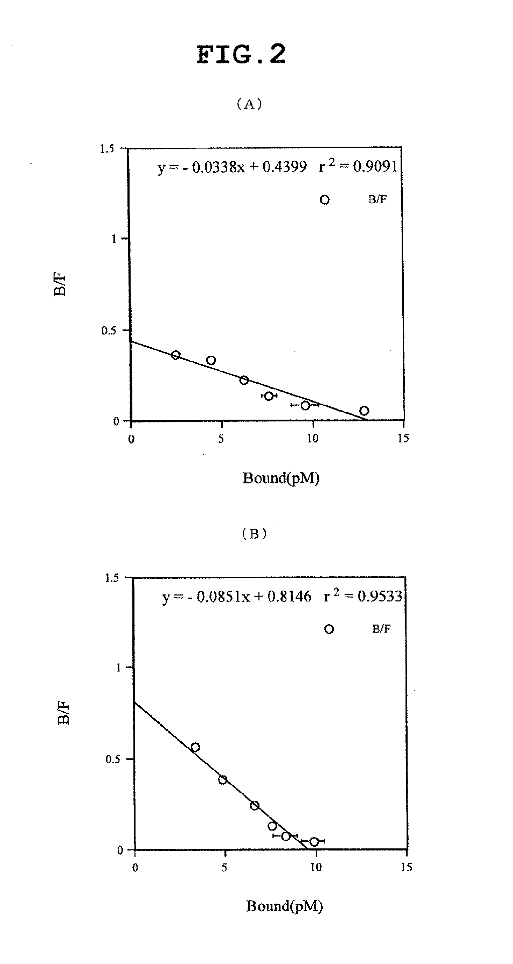

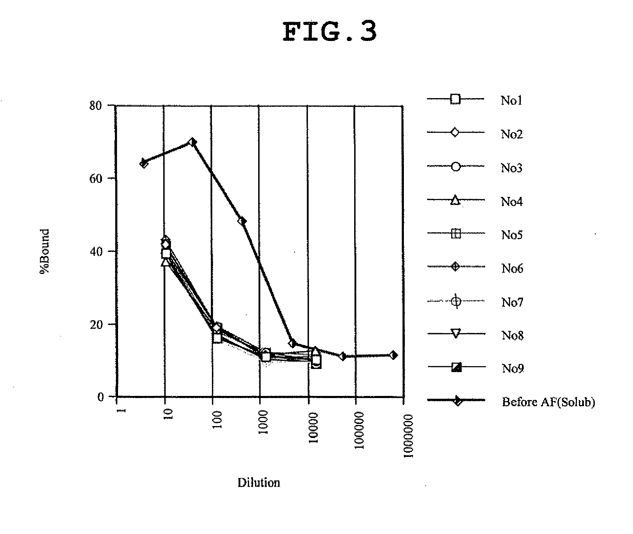

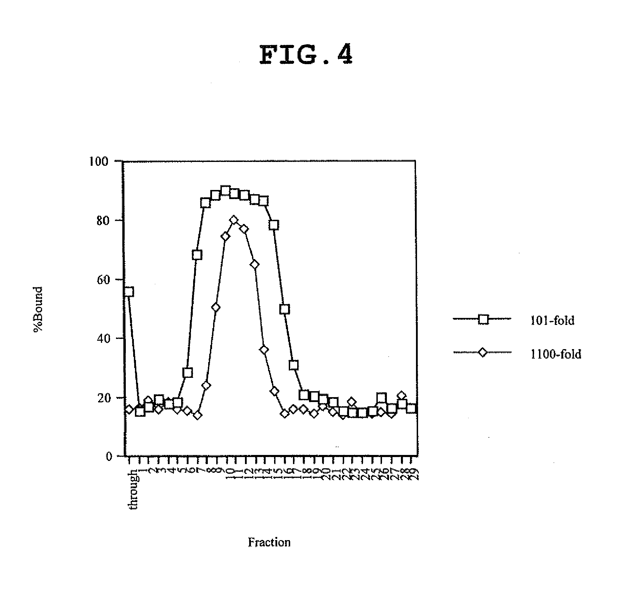

From the culture broth (14 L) of Sf9 cell expressing human PAC1, a membrane fraction was prepared by a Polytron homogenizer as usual. After dilution to a protein concentration of 2 mg/mL, the membrane fraction was solubilized by gentle rotation at 4°C in a solubilizing liquid containing 1.5% sucrose monolaulate (SM1200, Dojindo, S023) and 0.3% cholesteryl hemisuccinate Tris salt (CHS, Sigma, C6013). The apparent affinity of PAC1 before and after solubilization is shown in FIG. 1; Scatchard analyses of the membrane fraction and after solubilization are shown in FIG. 2. Because FIG. 1 plots the data with dilution rate indicated on the axis of abscissas relative to the 2 mg/mL membrane fraction used for the solubilization, the total affinity of receptor before and after solubilization of a given amount of the membrane fraction (proportional to the product of the number of receptors and equilibration constant (Kd)) is reflected. A comparison of the Scatchard analyses showed that 88% of the receptors of the membrane fraction was solubilized (FIG. 2).
Next, the solubilizing liquid was 3 fold diluted, after which 30 equivalents of Avidin-Affgel/Biotinyl PACAP38 was added, and affinity adsorption was performed (FIG. 3).
Thereafter, the affinity carrier was packed in a column, washed with about 900 mL, an amount 15 times the column volume, and about 5 mL of the eluate under acidic conditions was collected into a tube every 5 minutes. The affinities of the eluted fractions are shown in FIG. 4. Fractions 7 to 16 (about 70 mL including the washings) were collected, and protein was quantified using Amide Black to be 15 µg/mL.

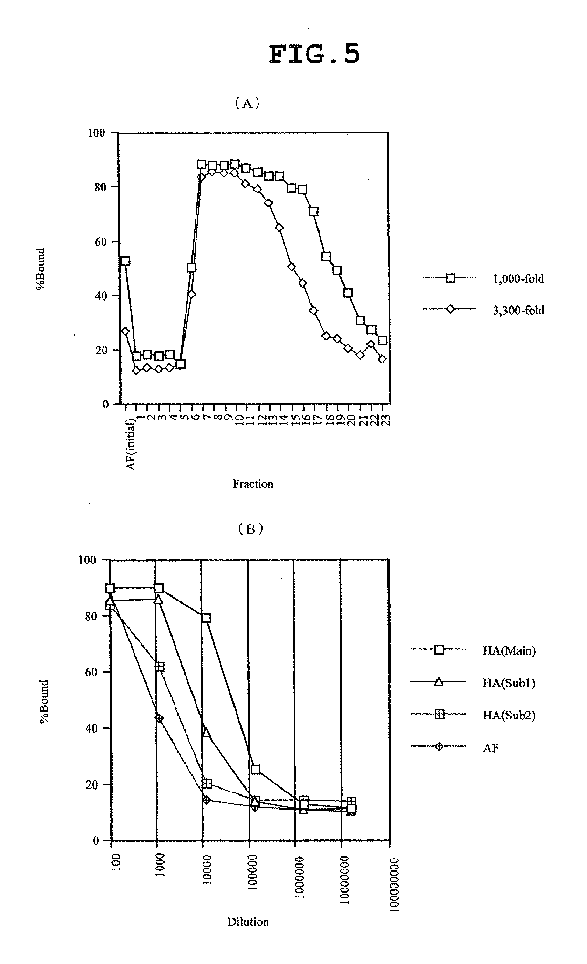

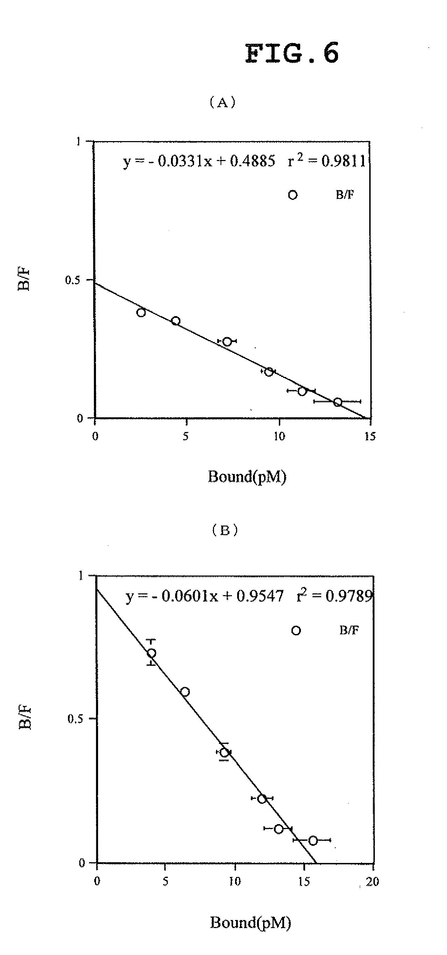

Subsequently, purification and concentration of PAC1 by hydroxyapatite chromatography was performed. 70 mL of the above-described affinity chromatography eluate (protein 1050 µg) was passed through a column 1 cm in inside diameter packed with 900 µL of hydroxyapatite carrier. The flow rate was set at about 6.6 mL/hr (range 6), the column was washed with about 9 mL, a volume 10 times the volume of the column, and the eluate was collected in an amount of about 0.44 mL every 4 minutes. The affinities of the eluted fractions are shown in FIG. 5A. Of these, fractions 7 to 11 were collected as the main fraction, fractions 12 to 14 were collected as the Sub1 fraction, and fraction 6 and 15 to 17 as the sub2 fraction; the apparent affinities thereof, along with the results for PAC1 receptor solution after affinity chromatography (AF(initial)), are shown in FIG. 5B. The Main fraction and the affinity chromatography eluted fraction were subjected to Scatchard analyses to analyze the characteristics of each fraction (FIG. 6). Thereby it was confirmed that human PAC1 was purified. The purified PAC1 receptor sample, for both the Main and subfractions, weighed about 1 mg.

### Example 2 Preparation of mouse anti-human PAC1 monoclonal antibody

With the PAC1 receptor purified in Example 1 above (hereinafter referred to as "SM1200/CHS solubilized PAC1 receptor") as an antigen, an emulsion prepared by mixing in a 1:1 ratio by volume the antigen and Freund's complete adjuvant for the first immunization, or Freund's incomplete adjuvant for the second and subsequent immunizations, was subcutaneously administered to BALB/C mice (6-week old, female) in an amount of 10 µg four times at 1-week intervals. After preliminary blood drawing, mice showing an increased antibody titer were selected, and each was subjected to final immunization (intravenous administration) with 10 µg of the antigen; 4 days later, the spleen was removed. Splenocytes were disassembled, thereafter filtered through sterile gauze, and suspended in TIL MediaI (manufactured by Immuno-Biological Laboratories Co., Ltd., Cat. No. 33612) containing 10% FCS, to yield a splenocyte suspension. The cell used for cell fusion was the BALB/C mouse derived myeloma cell P3×63.Ag8.653 (P3X63) (ATCC CRL1580). The cell fusion was performed by a modification of an original method (Nature, 256: 495, 1957). Hence, splenocytes and P3X63 were separately twice washed with serum-free TIL Media I, and mixed to obtain a ratio of splenocytes and P3X63 cells of 10:1, and centrifugation was performed at 1500 rpm for 5 minutes to precipitate the cells. After the supernatant was thoroughly removed, the precipitate was gently disassembled, 1.0 mL of 45%-polyethylene glycol (PEG)4000 (manufactured by Immuno-Biological Laboratories Co., Ltd., Cat. No. 33331) was added, and the mixture was allowed to stand in a 37°C warm water bath for 5 minutes to achieve fusion. After the fusion, TIL MediaI containing 10% FCS was added to the cells at a rate of 2 mL per minute; after a total of 40 mL of TIL Media I containing 10% FCS was added, the mixture was centrifuged at 800 rpm for 5 minutes, and the supernatant was removed. This cell precipitate was suspended in TIL MediaI containing 10% FCS to obtain a P3X63 density of 2×10⁶ cells per mL, and plated to a 96-well multidish (manufactured by Coaster) at 0.1 mL per well. After the plating, the cells were cultured in a CO₂ incubator at 37°C in an atmosphere of 5% CO₂/95% air. After 24 hours, HAT MediaI (Cat. No. 33251) containing 10% FCS and HAT (hypoxanthine 1×10⁻⁴ M, aminopterin 4×10⁻⁷ M, thymidine 1.6×10⁻⁵ M) (HAT medium) was added at 0.1 mL per well, whereby HAT selection culture was initiated. The HAT selection culture was continued while 0.1 mL of the old liquid was discarded 3, 6, and 9 days after the start of cultivation, after which 0.1 mL of HAT medium was added. Hybridoma proliferation was observed in 9 to 14 days after the cell fusion; when the culture broth turned into yellow (about 1×10⁶ cells/mL), the supernatant was collected and assayed for antibody titer.

### Example 3 Affinity of purified mouse anti-human PAC1 monoclonal antibody for antigen

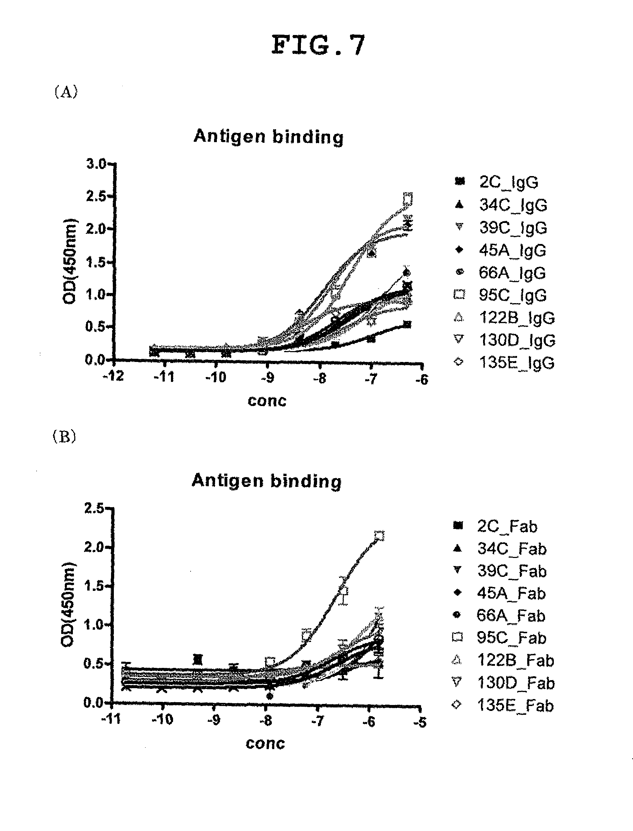

Antigen SM1200/CHS-solubilized PAC1 receptor was dispensed to MAXISORP at 50 µL/well to make 2 to 5 µg/mL, and allowed to stand overnight, after which 300 µL of a blocking solution containing 25% Block Ace was placed to cause blocking. After the plate was washed with PBS three times, solutions of the antibody obtained in Example 2 and the Fab of each antibody prepared by a conventional method were allowed to act at room temperature for 1.5 hours at various concentrations, thereafter a horseradish peroxidase (HRPO)-labeled goat antimouse IgG antibody (American Qualer, A106PU) in 1000-fold dilution was allowed to act at room temperature for 1 hour, and the plate was washed with a PBS containing 0.05% Tween 20 three times. Subsequently, 50 µL of HRPO substrate (TMB Peroxidase Substrate 2-component Kit, 50-76-00, manufactured by KPL) was dispensed to cause color development, and 50 µL of 1 N sulfuric acid was dispensed to stop the reaction, after which absorbance at 450 nm was measured using a plate reader (Multiscan; Labosystems). Results of the measurement are shown in FIG. 7; affinity data are shown in Table 1.

**[Table 1]**

| | Ag_Affinity | R² | Bmax | SubClass | chain |
|---|---|---|---|---|---|
| 2C | 1. 07E-07 | 0.9456 | 0.5153 | G1 | κ |
| 2C_Fab | 1. 48E-07 | 0.1093 | 0. 1215 | | |
| 34C | 3. 16E-08 | 0.9882 | 0.9414 | G1 | κ |
| 34C_Fab | 1. 83E-06 | 0.8953 | 1. 2961 | | |
| 39C | 1. 59E-08 | 0. 9917 | 2. 0118 | G2a | κ |
| 39C_Fab | 4. 00E-05 | 0.9494 | 23.4705 | | |
| 45A | 1. 12E-08 | 0.984 | 1. 8567 | G2a | κ |
| 45A_Fab | 3. 76E-07 | 0.8333 | 0.4229 | | |
| 66A | 1. 26E-07 | 0.9821 | 1. 5327 | G1 | κ |
| 66A_Fab | 1. 29E-06 | 0.8679 | 1. 4089 | | |
| 95C | 3. 95E-08 | 0.9725 | 2.3627 | G1 | κ |
| 95C_Fab | 2. 19E-07 | 0.9804 | 2.0456 | | |
| 122B | 7. 02E-08 | 0.9539 | 0.9774 | G1 | κ |
| 122B_Fab | 7. 48E-07 | 0.9658 | 1. 1969 | | |
| 130D | 2. 93E-08 | 0.9603 | 0. 7344 | G1 | κ |
| 130D_Fab | 2. 75E-07 | 0.9091 | 0.6949 | | |
| 135E | 4. 76E-09 | 0.9263 | 0. 7816 | G2a | κ |
| 135E_Fab | 1. 10E-08 | 0.6085 | 0.2396 | | |

### Identification of subclasses

Using the IsoStrip Mouse Monoclonal Antibody Isotyping Kit (Roche Diagnostics K.K., 1493027), the hybridoma culture supernatant was 30 fold diluted with D-PBS, added to a 150 µL tube, and evaluated. The subclasses of antibody clones were identified and shown in Table 1.

### Example 4 Inhibition of ligand binding of receptor by mouse anti-human PAC1 monoclonal antibodies

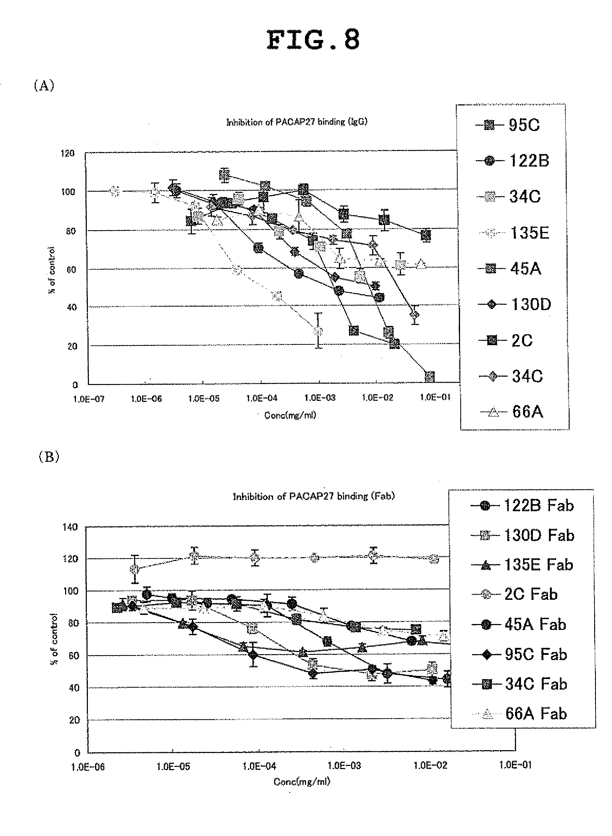

Purified SM1200/CHS-solubilized PAC1 receptor (0.4 mg/mL) was 200,000 fold diluted with TED-DG buffer (20 mM Tris-HCl (pH 7.5), 5 mM EDTA, 0.1% BSA, 0.05% Digitonin, 0.5 mM PMSF, 10 µg/mL Pepstatin A, 20 µg/mL Leupeptin, 4 µg/mL E-64), and 100 µL was dispensed to each 5 mL polyethylene tube (Falcon, 352053). A solution of the anti-PAC1 monoclonal antibody obtained in Example 2 in Dulbecco's PBS (D-PBS), 10 µL, was added at various concentrations, and this was followed by incubation for 15 to 30 minutes at 25°C, after which 5 µL of ¹²⁵I-PACAP27 (2 nM) was added, and this was followed by further incubation for 1 hour at 25°C. Subsequently, aspiration filtration was performed using a polyethyleneimine-treated GF/F glass filter (Whatman), and the radioactivity of ¹²⁵I-PACAP27 captured on the filter was detected using a γ counter. The measurement was performed three times. Taking a sample supplemented with D-PBS in place of the antibody as 100% bound, and one supplemented with cold PACAP27 to obtain a final concentration of 10⁻⁷ M as 0% bound, an evaluation was performed in terms of % of control. The results are shown in FIG. 8 and Table 2.

**[Table 2]**

| Anti-PAC1 mAb | IC₅₀ (M) |
|---|---|
| 2C | ND |
| 2C_Fab | ND |
| 34C | 3. 81E-08 |
| 34C_Fab | ND |
| 39C | ND |
| 39C_Fab | ND |
| 45A | NT |
| 45A_Fab | 1. 27E-07 |
| 66A | 8. 07E-08 |
| 66A_Fab | ND |
| 95C | 1. 28E-08 |
| 95C_Fab | 2. 16E-09 |
| 122B | 7. 68E-09 |
| 122B_Fab | 5. 01E-08 |
| 130D | 1. 48E-08 |
| 130D_Fab | 9. 33E-09 |
| 135E | 1. 00E-09 |
| 135E_Fab | 3. 99E-09 |

| | |
|---|---|
| ND: not detected NT: not tested | |

### Example 5 Inhibition of receptor activity of GTP-γS binding by mouse anti-human PAC1 monoclonal antibody

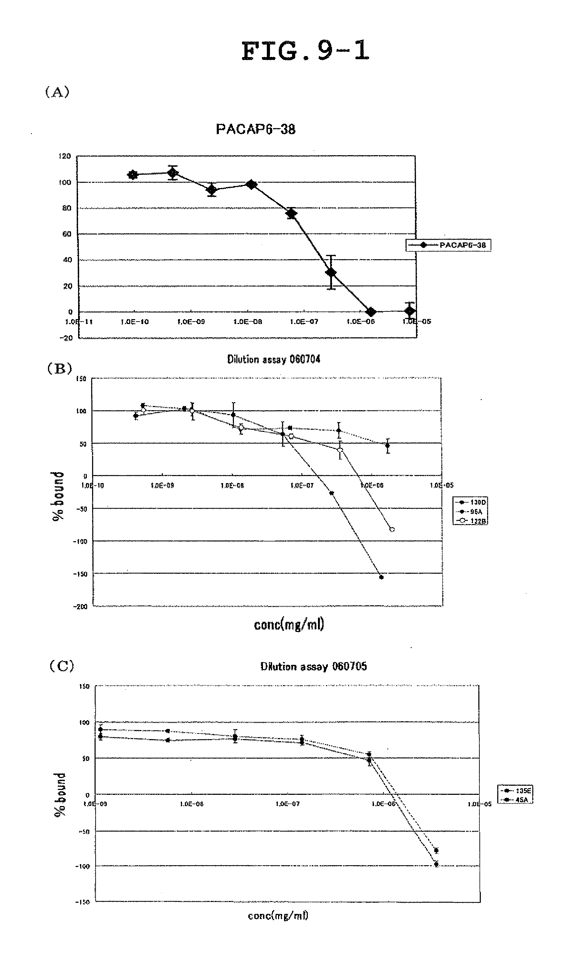

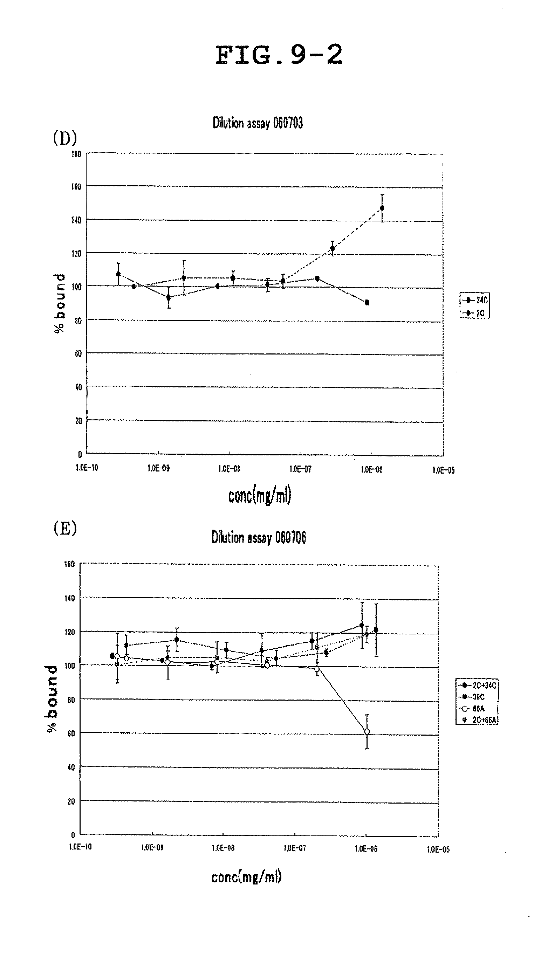

Chinese hamster ovary (CHO) cells having the PAC1 receptor gene introduced and expressed therein were disrupted by a Polytronhomogenizer, and 150 fold diluted with a buffer solution containing 20 mM Tris-HCl, 1 mM EDTA, 5 mM MgCl₂, 150 mM NaCl, 1 µM GDP, and 1% BSA to make 100 µL. 10 µL of the mouse anti-PAC1 monoclonal antibody obtained in Example 2 was added at various concentrations, and this was followed by incubation for 15 minutes at 25°C. Subsequently, 10 µl of 5 nM PACAP27 was added, and 10 µl of 10 nM GTPγ-³⁵S was further added, and this was followed by incubation for 60 minutes at 25°C. Subsequently, aspiration filtration was performed using a water-moistened GF/F glass filter (Whatman), and the filter was twice washed with 1.5 mL of a washing buffer solution containing 50 mM Tris-HCl (pH 7.4), 1 mM EDTA, 5 mM MgCl₂, 0.05% CHAPS, and 0.1% BSA. The radioactivity of GTPγ-³⁵S captured on the filter was detected using a liquid scintillator. The measurement was performed twice. Taking a sample supplemented with D-PBS in place of the antibody as 100% bound, and one supplemented with the antagonist PACAP6-38 to obtain a final concentration of 1.5×10⁻⁶ M as 0% bound, an evaluation was performed in terms of % of control. The results are shown in FIGS. 9-1 to 9-2.
In this system, the inhibition by the antagonist PACAP(6-38)NH₂ was estimated to be about IC₅₀ 10⁻⁷ M, a level about 50 times weaker than the reported value of 2 nM of IC₅₀ in the literature (Eur. J. Biochem. 207, 239 (1992)). The evaluation with this system showed that IgG 130D exhibited inhibition on the order of IC₅₀ 10⁻⁷ M, and 122B, 135E, 45A and 66A exhibited inhibition on the order of 10⁻⁶ M; of these, 130D, 122B, 135E and 45A are higher in the degree of inhibition of GTP-γS binding than the antagonist PACAP(6-38)NH₂.

### Example 6 Competitive ELISA with two mouse anti-human PAC1 monoclonal antibodies

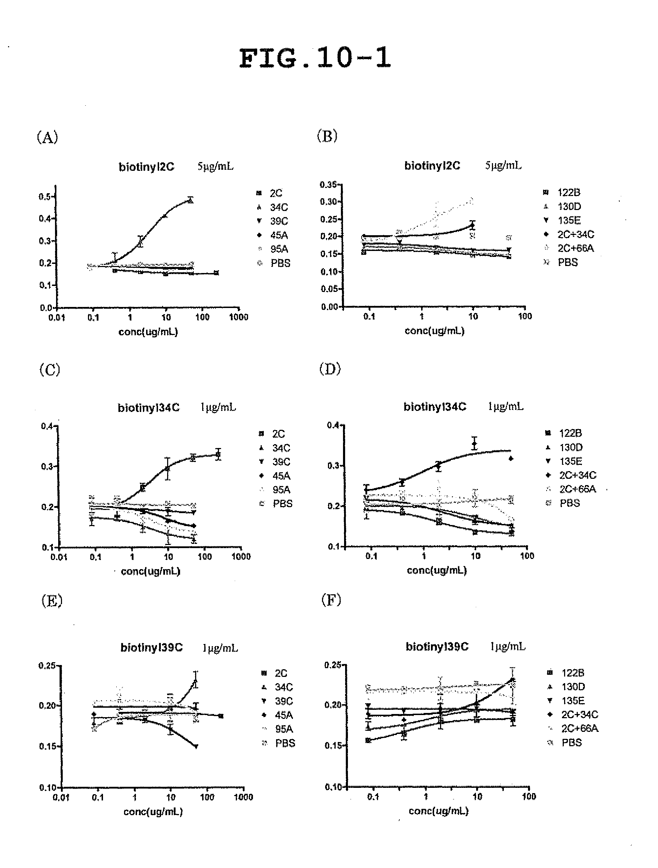

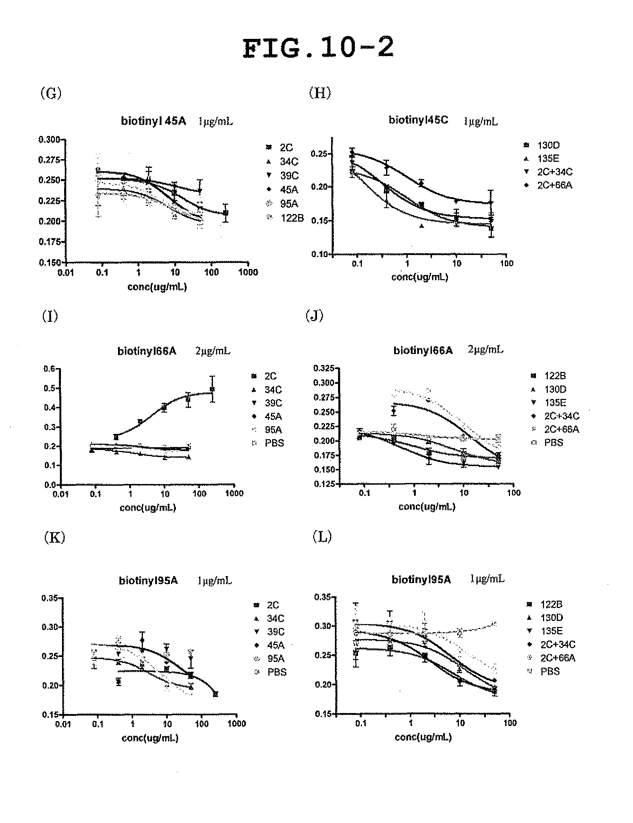

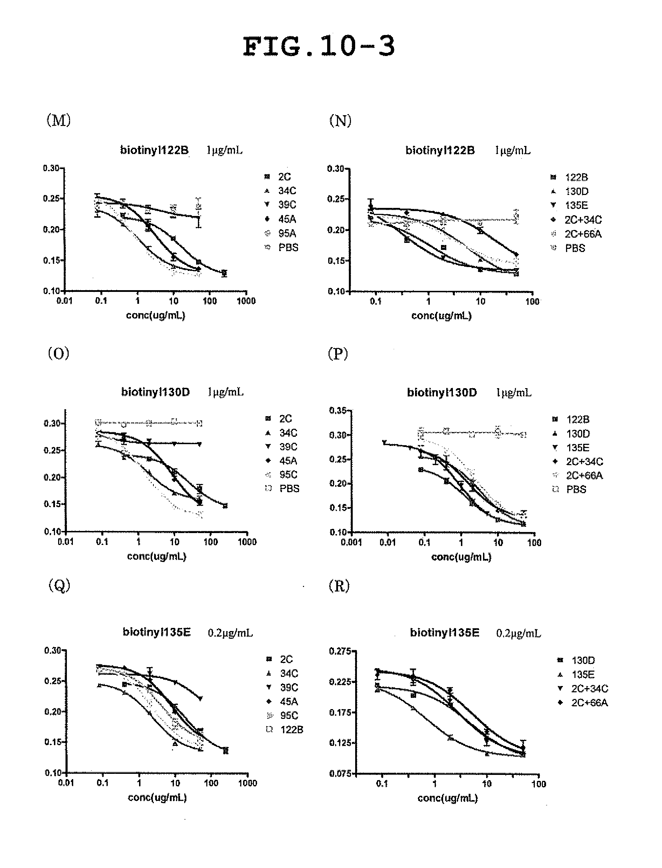

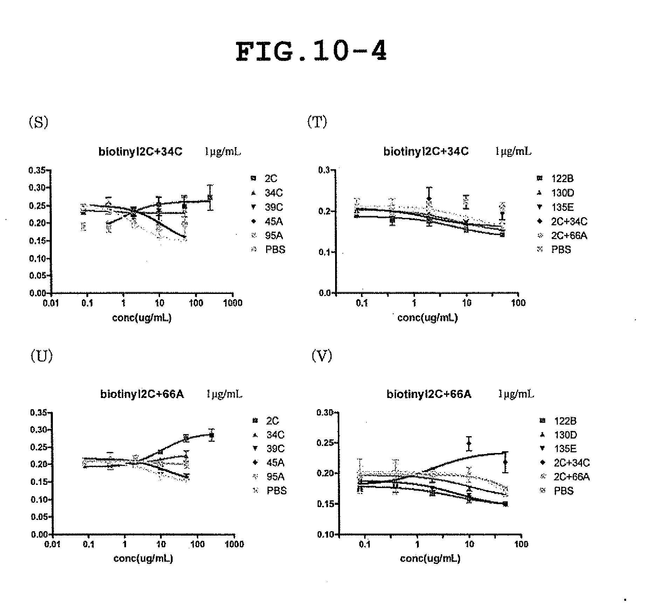

A solution of purified SM1200/CHS-solubilized PAC1 receptor, diluted to 2 µg/mL with 10 mM sodium phosphate buffer (pH 8.0) containing 100 mM NaCl, was added to a 96-well MAXSORP plate at 50 µL per well, and immobilized overnight. This plate was treated with 320 µL of a blocking solution (a PBS containing 25% Block Ace and 0.1% NaN₃) overnight to achieve blocking. After the plate was twice washed with PBS, the first mouse anti-human PAC1 monoclonal antibody obtained in Example 2, dissolved in PBS, was diluted with PBS in 4 levels or more, and 40 µl of each dilution was added (n=2). A second mouse anti-human PAC1 monoclonal antibody, biotinylated using a DOJINDO biotinylated kit (Biotin Labeling Kit - NH₂), was diluted with a buffer solution containing 20 mM sodium phosphate (pH 7.0), 0.4 M NaCl, 2 mM EDTA, 10% Block Ace, 0.2% BSA, and 0.05% CHAPS to a concentration of 1 µg/mL (except for 2C, 66A and 135E, which were diluted to 5 µg/mL, 2 µg/mL and 0.2 µg/mL, respectively), and 10 µL of each dilution was added (final concentration 0.2 µg/mL). After a reaction at room temperature for 2 hours, the plate was once washed with a D-PBS containing 0.05% Tween 20, and twice washed with D-PBS. Next, Avidin-HRP (Dako, P034) was 4000 fold diluted with 20 mM sodium phosphate (pH 7.0), 0.4 M NaCl, 2 mM EDTA, and 1.0% BSA; 50 µL of the dilution was added, and a reaction was carried out under shaking conditions at room temperature for 1.5 hours. After three times of washing with PBS containing 0.05% Tween 20, 50 µl of a 2-component system reagent was added to carry out an HRP reaction, and a color developing reaction was carried out for 3 to 7 minutes, after which 50 µL of H₂SO₄ was added to stop the reaction, and a colorimetric quantitation was performed at 450 nm using a plate reader. The results are shown in FIGS. 10-1 to 10-4 and Table 3.

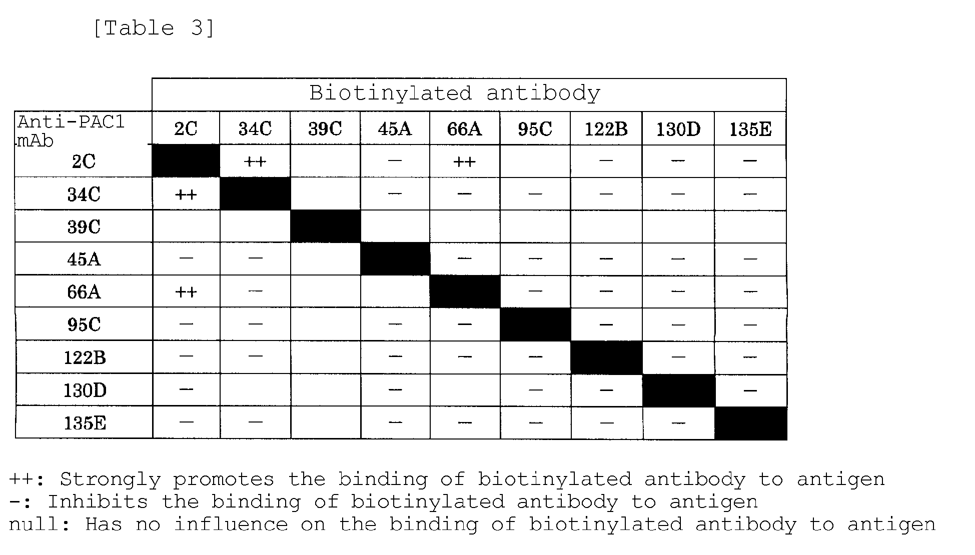

In this measurement, the competition between the same biotinylated and non-labeled antibodies was evaluated for positive control, and a combination displaced in the same dynamic range as the positive control was judged to inhibit each other ("-" in Table 3). When this system was established, it was thought that only two different results would be obtained, i.e., this result (-) and no inhibition of binding of biotinylated antibody (null). However, conversely, with the addition of non-labeled antibody, binding of biotinylated antibody was strongly promoted (++) in some cases. In particular, IgG-2C and IgG-34C, and IgG-2C and IgG-66A, when co-existing with each other, promoted the mutual binding. Meanwhile, IgG-34C and IgG-66A mutually inhibited the binding thereof. Six kinds, i.e., IgG-2C, IgG-45A, IgG-95C, IgG-122B, IgG-130D and IgG-135E, inhibited each other, and were therefore found to have mutually overlapping antigen binding sites. The antigen binding sites of these antibodies are thought to be extracellularly exposed regions of PAC1. IgG-39C, which does not bind to receptors expressed on the cell surface, did not inhibit or promote the binding to other antibodies.

### [Industrial Applicability]

The anti-PAC1 antibody of the present invention is highly useful as a prophylactic/therapeutic agent for a disease associated with accentuation of a bioactivity of PAC1, and a diagnostic reagent for a disease associated with an abnormality of PAC1 activity, because it is capable of regulating PAC1 activity. The antibody of the present invention is also useful in screening for a substance that regulates the expression of PAC1.

While the present invention has been described with emphasis on preferred embodiments, it is obvious to those skilled in the art that the preferred embodiments can be modified. The present invention intends that the present invention can be embodied by methods other than those described in detail in the present specification. Accordingly, the present invention encompasses all modifications encompassed in the gist and scope of the appended "CLAIMS".
This application is based on patent application No. 2006-216282 (filing date: August 8, 2006) filed in Japan, and the contents disclosed therein are hereby entirely incorporated by reference. In addition, the contents disclosed in any publication cited herein, including patents and patent applications, are hereby incorporated in their entireties by reference, to the extent that they have been disclosed herein.

## Claims

1. An anti-PAC1 monoclonal antibody capable of recognizing a PAC1 having a native structure.

2. The antibody of claim 1, which recognizes an extracellular region of PAC1.

3. The antibody of claim 2, which inhibits the binding of PAC1 to PACAP.

4. The antibody of claim 3, which inhibits a bioactivity of PAC1.

5. The antibody of claim 2, which does not inhibit the binding of PAC1 to PACAP.

6. The antibody of claim 5, which promotes the binding of another anti-PAC1 monoclonal antibody to PAC1.

7. The antibody of claim 6, wherein the other anti-PAC1 monoclonal antibody inhibits the binding of PAC1 to PACAP.

8. The antibody of claim 1, which is further capable of recognizing denatured PAC1.

9. A PAC1 activity regulator comprising the antibody of claim 1.

10. A PAC1 activity inhibitor comprising the antibody of claim 4.

11. The agent of claim 10, which is to be used for the prevention/treatment of a disease associated with accentuation of a bioactivity of PAC1.

12. A PAC1 detection agent comprising the antibody of claim 1.

13. The agent of claim 12, which is to be used for the diagnosis of a disease associated with an abnormality of PAC1 activity.

14. A screening method for a substance that regulates the expression of PAC1, comprising using the antibody of claim 1 and a PAC1-expressing cell.
